# EUROPÄISCHE PATENTSCHRIFT

(11) **EP 1 737 842 B1**
(45) Veröffentlichungstag und Bekanntmachung des Hinweises auf die Patenterteilung: **18.08.2010**
(21) Anmeldenummer: 05731650.7
(22) Anmeldetag: 09.04.2005
(51) Int. Cl.: C07D 401/14, C07D 471/10, C07D 513/04, C07D 453/04, C07D 401/04, C07D 451/04, C07D 471/04, C07D 495/04, C07D 487/08, A61K 31/4545, A61P 25/06

(54) **AUSGEWÄHLTE CGRP-ANTAGONISTEN, VERFAHREN ZU DEREN HERSTELLUNG SOWIE DEREN VERWENDUNG ALS ARZNEIMITTEL**
SELECTED CGRP ANTAGONISTS, METHOD FOR PRODUCING THE SAME AND THE USE THEREOF AS DRUGS
ANTAGONISTES CGRP SELECTIONNES, LEUR PROCEDE DE PRODUCTION ET LEUR UTILISATION COMME MEDICAMENTS

(30) Priorität: 15.04.2004 DE 102004018795
(43) Veröffentlichungstag der Anmeldung: 03.01.2007
(73) Patentinhaber: Boehringer Ingelheim International GmbH, 55216 Ingelheim am Rhein (DE); Boehringer Ingelheim Pharma GmbH & Co. KG, 55216 Ingelheim (DE)
(72) Erfinder: MUELLER, Stephan Georg, 88447 Warthausen (DE); RUDOLF, Klaus, 88447 Warthausen (DE); LUSTENBERGER, Philipp, 4056 Basel (CH); STENKAMP, Dirk, 88400 Biberach (DE); ARNDT, Kirsten, 88400 Biberach (DE); DOODS, Henri, 88447 Warthausen (DE); SCHAENZLE, Gerhard, 88400 Biberach-Mettenberg (DE)
(74) Vertreter: Hammann, Heinz
(86) Internationale Anmeldenummer: PCT/EP2005/003741
(87) Internationale Veröffentlichungsnummer: WO 2005/100343

(56) Entgegenhaltungen:
- WO-A-01/32649
- WO-A-03/070753
- WO-A1-98/11128
- WO-A1-2004/037810
- WO-A1-2004/037811
- WO-A1-2004/063171
- WO-A2-2004/000289

## Beschreibung

Gegenstand der vorliegenden Erfindung sind einzelne CGRP-Antagonisten der allgemeinen Formel 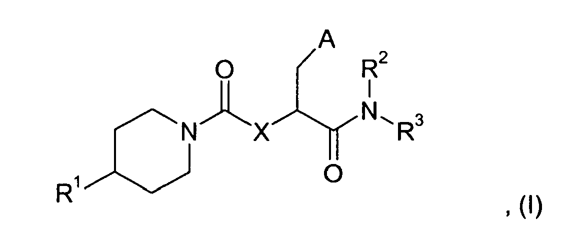 deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren, diese Verbindungen enthaltende Arzneimittel, deren Verwendung und Verfahren zu ihrer Herstellung.

Im Stand der Technik werden in der WO 2004/037810, WO 98/11128, WO 01/32649, WO 2004/063171 und WO 2004/037811 bereits CGRP-Antagonisten beschrieben, die sich jedoch strukturell von denen der vorliegenden Anmeldung unterscheiden.

In der obigen allgemeinen Formel (I) bedeuten

A einen Rest der Formel 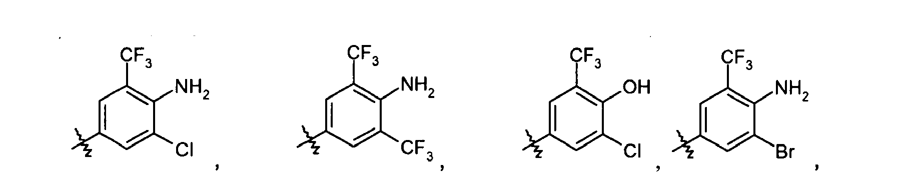 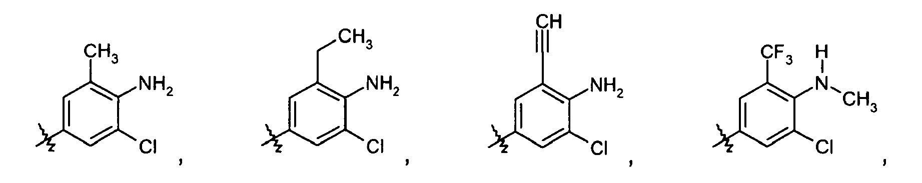 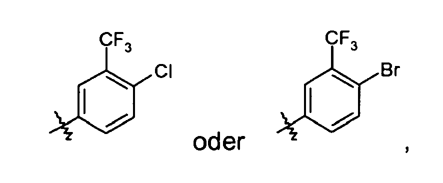 X ein Sauerstoffatom, eine Methylen- oder NH-Gruppe,
R¹ einen Rest der Formel 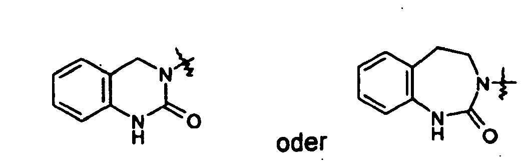 und
-NR²R³ einen Rest der Formel

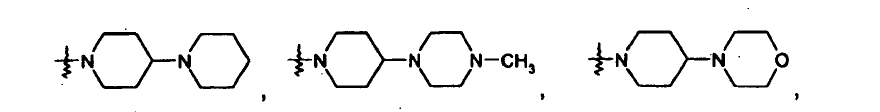

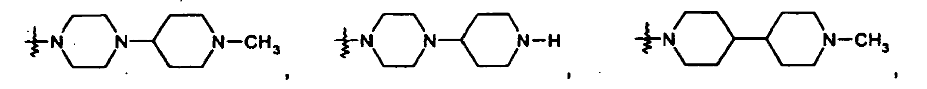

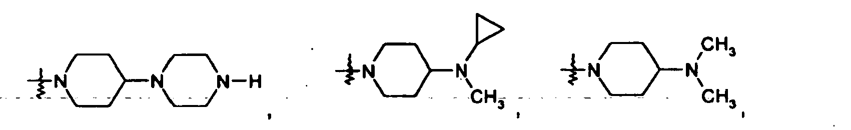

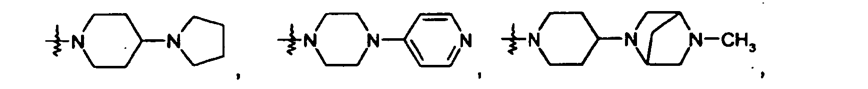

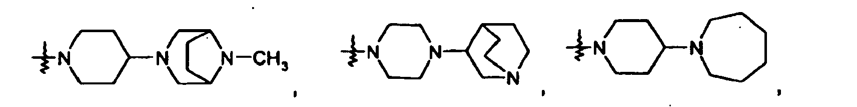

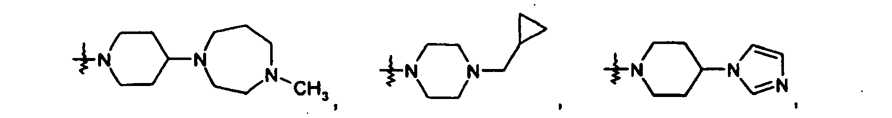

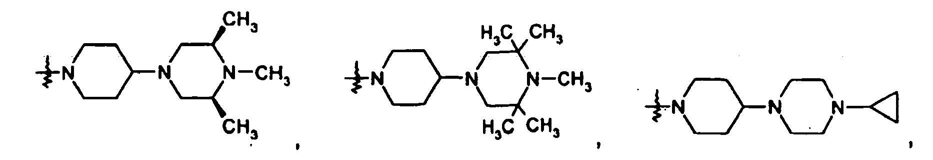

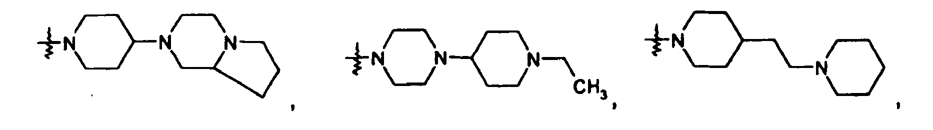

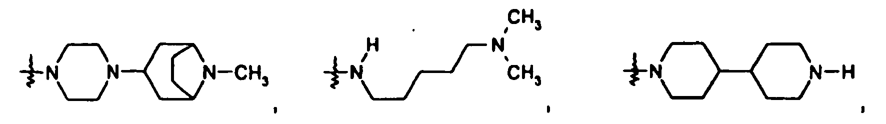

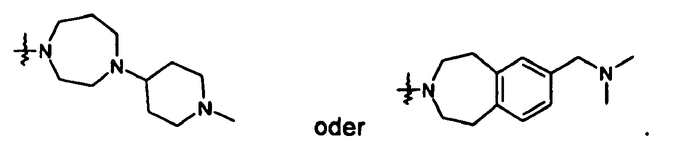

Besonders bevorzugte Verbindungen der obigen allgemeinen Formel (I) sind beispielsweise folgende:
(1) 4-(2-Oxo-1,2,4,5-tetrahydro-benzo[*d*][1,3]diazepin-3-yl)-piperidin-1-carbonsäure-(*R*)-1-(4-amino-3-chlor-5-trifluormethyl-benzyl)-2-oxo-2-[4-(2-piperidin-1-yl-ethyl)-piperidin-1-yl]-ethylester,
(2) 4-(2-Oxo-1,2,4,5-tetrahydro-benzo[*d*][1,3]diazepin-3-yl)-piperidin-1-carbonsäure-(*R*)-1-(4-amino-3-chlor-5-trifluormethyl-benzyl)-2-[4-(1-aza-bicyclo-[2.2.2]oct-3-yl)-piperazin-1-yl]-2-oxo-ethylester,
(3) 4-(2-Oxo-1,2,4,5-tetrahydro-benzo[*d*][1,3]diazepin-3-yl)-piperidin-1-carbonsäure-(*R*)-1-(4-amino-3-chlor-5-trifluormethyl-benzyl)-2-[4-(5-methyl-2,5-diazabicyclo[2.2.1]hept-2-yl)-piperidin-1-yl]-2-oxo-ethylester,
(4) 4-(2-Oxo-1,2,4,5-tetrahydro-benzo[*d*][1,3]diazepin-3-yl)-piperidin-1-carbonsäure-(*R*)-2-(4-amino-3-chlor-5-trifluormethyl-phenyl)-1-(5-dimethylaminopentylcarbamoyl)-ethylester,
(5) 4-(2-Oxo-1,2,4,5-tetrahydro-benzo[*d*][1,3]diazepin-3-yl)-piperidin-1-carbonsäure-(*R*)-1-(4-amino-3-chlor-5-trifluormethyl-benzyl)-2-oxo-2-[4-((3*R*,5*S*)-3,4,5-trimethyl-piperazin-1-yl)-piperidin-1-yl]-ethylester,
(6) 4-(2-Oxo-1,2,4,5-tetrahydro-benzo[*d*][1,3]diazepin-3-yl)-piperidin-1-carbonsäure-(*R*)-1-(4-amino-3-chlor-5-trifluormethyl-benzyl)-2-oxo-2-[4-(3,3,4,5,5-pentamethyl-piperazin-1-yl)-piperidin-1-yi]-ethylester,
(7) 4-(2-Oxo-1,2,4,5-tetrahydro-benzo[*d*][1,3]diazepin-3-yl)-piperidin-1-carbonsäure-(*R*)-1-(4-amino-3-chlor-5-trifluormethyl-benzyl)-2-[4-(4-cyclopropylpiperazin-1-yl)-piperidin-1-yl]-2-oxo-ethylester,
(8) 4-(2-Oxo-1,2,4,5-tetrahydro-benzo[*d*][1,3]diazepin-3-yl)-piperidin-1-carbonsäure-(*R*)-1-(4-amino-3-chlor-5-trifluormethyl-benzyl)-2-[4-(1-methyl-piperidin-4-yl)-[1,4]diazepan-1-yl]-2-oxo-ethylester,
(9) 4-(2-Oxo-1,2,4,5-tetrahydro-benzo[*d*][1,3]diazepin-3-yl)-piperidin-1-carbonsäure-(*R*)-1-(4-amino-3-chlor-5-trifluormethyl-benzyl)-2-(7-dimethylaminomethyl-1,2,4,5-tetrahydro-benzo[*d*]azepin-3-yl)-2-oxo-ethylester,
(10) 4-(2-Oxo-1,2,4,5-tetrahydro-benzo[*d*][1,3]diazepin-3-yl)-piperidin-1-carbonsäure-(*R*)-1-(4-amino-3-chlor-5-trifluormethyl-benzyl)-2-[4-(cyclopropyl-methyl-amino)-piperidin-1-yl]-2-oxo-ethylester,
(11) 4-(2-Oxo-1,2,4,5-tetrahydro-benzo[*d*][1,3]diazepin-3-yl)-piperidin-1-carbonsäure-(*R*)-1-(4-amino-3-chlor-5-trifluormethyl-benzyl)-2-[4-(hexahydro-pyrrolo-[1,2-a]pyrazin-2-yl)-piperidin-1-yl]-2-oxo-ethylester,
(12) 4-(2-Oxo-1,2,4,5-tetrahydro-benzo[*d*][1,3]diazepin-3-yl)-piperidin-1-carbonsäure-(*R*)-1-(4-amino-3-chlor-5-trifluormethyl-benzyl)-2-[4-(1-ethyl-piperidin-4-yl)-piperazin-1-yl]-2-oxo-ethylester,
(13) 4-(2-Oxo-1,2,4,5-tetrahydro-benzo[*d*][1,3]diazepin-3-yl)-piperidin-1-carbonsäure-(*R*)-1-(4-amino-3-chlor-5-trifluormethyl-benzyl)-2-[4-(8-methyl-8-azabicyclo[3.2.1]oct-3-yl)-piperazin-1-yl]-2-oxo-ethylester.
(14) 4-(2-Oxo-1,2,4,5-tetrahydro-benzo[*d*][1,3]diazepin-3-yl)-piperidin-1-carbonsäure-(*R*)-1-(4-amino-3-chlor-5-trifluormethyl-benzyl)-2-[4-(4-methyl-[1,4]diazepan-1-yl)-piperidin-1-yl]-2-oxo-ethylester,
(15) 4-(2-Oxo-1,2,4,5-tetrahydro-benzo[*d*][1,3]diazepin-3-yl)-piperidin-1-carbonsäure-(*R*)-1-(4-amino-3-chlor-5-trifluormethyl-benzyl)-2-(4-cyclopropylmethyl-piperazin-1-yl)-2-oxo-ethylester,
(16) 4-(2-Oxo-1,2,4,5-tetrahydro-benzo[*d*][1,3]diazepin-3-yl)-piperidin-1-carbonsäure-(*R*)-1-(4-amino-3-chlor-5-trifluormethyl-benzyl)-2-(4-azepan-1-yl-piperidin-1-yl)-2-oxo-ethylester,
(17) 4-(2-Oxo-1,2,4,5-tetrahydro-benzo[*d*][1,3]diazepin-3-yl)-piperidin-1-carbonsäure-(*R*)-1-(4-amino-3-chlor-5-trifluormethyl-benzyl)-2-(4-morpholin-4-yl-piperidin-1-yl)-2-oxo-ethylester,
(18) 4-(2-Oxo-1,2,4,5-tetrahydro-benzo[*d*][1,3]diazepin-3-yl)-piperidin-1-carbonsäure-(*R*)-1-(4-amino-3-chlor-5-trifluormethyl-benzyl)-2-(4-imidazol-1-yl-piperid in-1-yl)-2-oxo-ethylester,
(19) 4-(2-Oxo-1,2,4,5-tetrahydro-benzo[*d*][1,3]diazepin-3-yl)-piperidin-1-carbonsäure-(*R*)-1-(4-chtor-3-trifluormethyl-benzyl)-2-[4-(4-methyl-piperazin-1-yl)-piperidin-1-yl]-2-oxo-ethylester,
(20) 4-(2-Oxo-1,2,4,5-tetrahydro-benzo[*d*][1,3]diazepin-3-yl)-piperidin-1-carbonsäure-(*R*)-1-(4-chlor-3-trifluormethyl-benzyl)-2-[4-(1-methyl-piperidin-4-yl)-piperazin-1-yl]-2-oxo-ethylester,
deren Tautomeren, deren Diastereomeren, deren Enantiomeren, deren Hydraten, deren Gemischen und deren Salzen sowie den Hydraten der Salze eine herausragende Bedeutung zukommt.

Die erfindungsgemäßen neuen Verbindungen der allgemeinen Formel (I) enthalten ein oder mehrere Chiralitätszentren. Sind beispielsweise zwei Chiralitätszentren vorhanden, dann können die Verbindungen in Form zweier diastereomerer Antipodenpaare auftreten. Die Erfindung umfasst die einzelnen Isomeren ebenso wie ihre Gemische.

Die Trennung der jeweiligen Diastereomeren gelingt auf Grund ihrer unterschiedlichen physikochemischen Eigenschaften, z.B. durch fraktionierte Kristallisation aus geeigneten Lösemitteln, durch Hochdruckflüssigkeits- oder Säulenchromatographie unter Verwendung chiraler oder bevorzugt achiraler stationärer Phasen.

Die Trennung von unter die allgemeine Formel (I) fallenden Racematen gelingt beispielsweise durch HPLC an geeigneten chiralen stationären Phasen (z. B. Chiral AGP, Chiralpak AD). Racemate, die eine basische Funktion enthalten, lassen sich auch über die diastereomeren, optisch aktiven Salze trennen, die bei Umsetzung mit einer optisch aktiven Säure, beispielsweise (+)- oder (-)-Weinsäure, (+)- oder (-)-Diacetylweinsäure, (+)- oder (-)-Monomethyltartrat oder (+)-Camphersulfonsäure entstehen.

Nach einem üblichen Verfahren zur Isomerentrennung wird das Racemat einer Verbindung der allgemeinen Formel (I) mit einer der vorstehend angegebenen optisch aktiven Säuren in äquimolarer Menge in einem Lösemittel ungesetzt und die erhaltenen kristallinen, diastereomeren, optisch aktiven Salze unter Ausnutzung ihrer verschiedenen Löslichkeit getrennt. Diese Umsetzung kann in jeder Art von Lösemitteln durchgeführt werden, solange sie einen ausreichenden Unterschied hinsichtlich der Löslichkeit der Salze aufweisen. Vorzugsweise werden Methanol, Ethanol oder deren Gemische, beispielsweise im Volumenverhältnis 50:50, verwendet. Sodann wird jedes der optisch aktiven Salze in Wasser gelöst, mit einer Base, wie Natriumcarbonat oder Kaliumcarbonat, oder mit einer geeigneten Säure, beispielsweise mit verdünnter Salzsäure oder wässeriger Methansulfonsäure, vorsichtig neutralisiert und dadurch die entsprechende freie Verbindung in der (+)- oder (-)-Form erhalten.

Jeweils nur das (R)- oder (S)-Enantiomer bzw. ein Gemisch zweier optisch aktiver, unter die allgemeine Formel (I) fallender diastereomerer Verbindungen wird auch dadurch erhalten, dass man die oben beschriebenen Synthesen mit jeweils einer geeigneten (R)- bzw. (S)-konfigurierten Reaktionskomponente durchführt.

Die erhaltenen Verbindungen der allgemeinen Formel (I) können, sofern sie geeignete basische Funktionen enthalten, insbesondere für pharmazeutische Anwendungen in ihre physiologisch verträglichen Salze mit anorganischen oder organischen Säuren übergeführt werden. Als Säuren kommen hierfür beispielsweise Salzsäure, Bromwasserstoffsäure, Phosphorsäure, Salpetersäure, Schwefelsäure, Methansulfonsäure, Ethansulfonsäure, Benzolsulfonsäure, *p*-Toluolsulfonsäure, Essigsäure, Fumarsäure, Bernsteinsäure, Milchsäure, Mandelsäure, Äpfelsäure, Zitronensäure, Weinsäure oder Maleinsäure in Betracht.

Die vorliegende Erfindung betrifft Racemate, sofern die Verbindungen der allgemeinen Formel (I) nur ein Chiralitätselement besitzen. Die Anmeldung umfasst jedoch auch die einzelnen diastereomeren Antipodenpaare oder deren Gemische, die dann vorliegen, wenn mehr als ein Chiralitätselement in den Verbindungen der allgemeinen Formel (I) vorhanden ist, sowie die einzelnen optisch aktiven Enantiomeren, aus denen sich die erwähnten Racemate zusammensetzen.

Ebenfalls mit vom Gegenstand dieser Erfindung umfasst sind die erfindungsgemäßen Verbindungen, einschließlich deren Salze, in denen ein oder mehrere Wasserstoffatome durch Deuterium ausgetauscht sind.

Die neuen Verbindungen der allgemeinen Formel (I) und deren physiologisch verträgliche Salze weisen wertvolle pharmakotogische Eigenschaften auf, die auf ihre selektiven CGRP-antagonistischen Eigenschaften zurückgehen. Ein weiterer Gegenstand der Erfindung sind diese Verbindungen enthaltende Arzneimittel, deren Verwendung und deren Herstellung.

Die voranstehend genannten neuen Verbindungen und deren physiologisch verträgliche Salze besitzen CGRP-antagonistische Eigenschaften und zeigen gute Affinitäten in CGRP-Rezeptorbindungsstudien. Die Verbindungen weisen in den nachstehend beschriebenen pharmakologischen Testsystemen CGRP-antagonistische Eigenschaften auf.

Zum Nachweis der Affinität der voranstehend genannten Verbindungen zu humanen CGRP-Rezeptoren und ihrer antagonistischen Eigenschaften wurden die folgenden Versuche durchgeführt:

### A. Bindungsstudien mit (den humanen CGRP-Rezeptor exprimierenden) SK-N-MC-Zellen

SK-N-MC-Zellen werden in "Dulbecco's modified Eagle Medium" kultiviert. Das Medium konfluenter Kulturen wird entfernt. Die Zellen werden zweimal mit PBS-Puffer (Gibco 041-04190 M) gewaschen, durch Zugabe von PBS-Puffer, versetzt mit 0.02% EDTA, abgelöst und durch Zentrifugation isoliert. Nach Resuspension in 20 ml "Balanced Salts Solution" [BSS (in mM): NaCl 120, KCI 5.4, NaHCO₃ 16.2, MgSO₄ 0.8, NaHPO₄ 1.0, CaCl₂ 1.8, D-Glucose 5.5, HEPES 30, pH 7.40] werden die Zellen zweimal bei 100 x g zentrifugiert und in BSS resuspendiert. Nach Bestimmung der Zellzahl werden die Zellen mit Hilfe eines Ultra-Turrax homogenisiert und für 10 Minuten bei 3000 x g zentrifugiert. Der Überstand wird verworfen und das Pellet in Tris-Puffer (10 mM Tris, 50 mM NaCl, 5 mM MgCl₂, 1 mM EDTA, pH 7.40), angereichert mit 1 % Rinderserum-Albumin und 0.1% Bacitracin, rezentrifugiert und resuspendiert (1 ml / 1000000 Zellen). Das Homogenat wird bei -80°C eingefroren. Die Membranpräparationen sind bei diesen Bedingungen für mehr als 6 Wochen stabil.

Nach Auftauen wird das Homogenat 1:10 mit Assay-Puffer (50 mM Tris, 150 mM NaCl, 5 mM MgCl₂, 1 mM EDTA, pH 7.40) verdünnt und 30 Sekunden lang mit einem Ultra-Turrax homogenisiert. 230 µl des Homogenats werden für 180 Minuten bei Raumtemperatur mit 50 pM ¹²⁵I-lodtyrosyl-Calcitonin-Gene-Related Peptide (Amersham) und ansteigenden Konzentrationen der Testsubstanzen in einem Gesamtvolumen von 250 µl inkubiert. Die Inkubation wird durch rasche Filtration durch mit Polyethylenimin (0.1%) behandelte GF/B-Glasfaserfilter mittels eines Zellharvesters beendet. Die an Protein gebundene Radioaktivität wird mit Hilfe eines Gammacounters bestimmt. Als nichtspezifische Bindung wird die gebundene Radioaktivität nach Gegenwart von 1 µM humanem CGRP-alpha während der Inkubation definiert.

Die Analyse der Konzentrations-Bindungskurven erfolgt mit Hilfe einer computergestützten nichtlinearen Kurvenanpassung.

Die eingangs erwähnten Verbindungen zeigen in dem beschriebenen Test IC₅₀-Werte ≤ 10000 nM.

### B. CGRP-Antagonismus in SK-N-MC-Zellen

SK-N-MC-Zellen (1 Mio. Zellen) werden zweimal mit 250 µl Inkubationspuffer (Hanks' HEPES, 1 mM 3-lsobutyl-1-methylxanthin, 1% BSA, pH 7.4) gewaschen und bei 37°C für 15 Minuten vorinkubiert. Nach Zugabe von CGRP (10 µl) als Agonist in steigenden Konzentrationen (10⁻¹¹ bis 10⁻⁶ M) bzw. zusätzlich von Substanz in 3 bis 4 verschiedenen Konzentrationen wird nochmals 15 Minuten inkubiert.

Intrazelluläres cAMP wird anschließend durch Zugabe von 20 µl 1M HCl und Zentrifugation (2000 x g, 4°C für 15 Minuten) extrahiert. Die Überstände werden in flüssigem Stickstoff eingefroren und bei 20°C gelagert.

Die cAMP-Gehalte der Proben werden mittels Radioimmunassay (Fa. Amersham) bestimmt und die pA₂-Werte antagonistisch wirkender Substanzen graphisch ermittelt.

Die erfindungsgemäßen Verbindungen zeigen in dem beschriebenen *in vitro* Testmodell CGRP-antagonistische Eigenschaften in einem Dosisbereich zwischen 10⁻¹² bis 10⁻⁵ M.

Aufgrund ihrer pharmakologischen Eigenschaften eignen sich die erfindungsgemäßen Verbindungen und deren Salze mit physiologisch verträglichen Säuren somit zur akuten und prophylaktischen Behandlung von Kopfschmerzen, insbesondere Migräne- bzw. Cluster-Kopfschmerz. Weiterhin beeinflussen die erfindungsgemäßen Verbindungen auch die folgenden Erkrankungen positiv:
Nicht-insulinabhängigen Diabetes mellitus ("NIDOM"), complex regional pain syndrome (CRPS1), cardiovaskuläre Erkrankungen, Morphintoleranz, Clostritiumtoxin-bedingte Durchfallerkrankungen, Erkrankungen der Haut, insbesondere thermische und strahlenbedingte Hautschäden inklusive Sonnenbrand, entzündliche Erkrankungen, z.B. entzündliche Gelenkerkrankungen (Arthritis), neurogene Entzündungen der oralen Mucosa, entzündliche Lungenerkrankungen, allergische Rhinitis, Asthma, Erkrankungen, die mit einer überschießenden Gefäßerweiterung und dadurch bedingter verringerter Gewebedurchblutung einhergehen, z.B. Schock und Sepsis. Darüber hinaus zeigen die erfindungsgemäßen Verbindungen eine lindernde Wirkung auf Schmerzzustände im allgemeinen.

Die Symptomatik menopausaler, durch Gefäßerweiterung und erhöhten Blutfluss verursachter Hitzewallungen östrogendefizienter Frauen sowie hormonbehandelter Prostatakarzinompatienten wird durch die CGRP-Antagonisten der vorliegenden Anwendung präventiv und akut-therapeutisch günstig beeinflusst, wobei sich dieser Therapieansatz vor der Hormonsubstitution durch Nebenwirkungsarmut auszeichnet.

Die zur Erzielung einer entsprechenden Wirkung erforderliche Dosierung beträgt zweckmäßigerweise bei intravenöser oder subkutaner Gabe 0.01 bis 3 mg/kg Körpergewicht, vorzugsweise 0.01 bis 1 mg/kg Körpergewicht, bei oraler Gabe 0.01 bis 20 mg/kg Körpergewicht, vorzugsweise 0.1 bis 10 mg/kg Körpergewicht, und bei nasaler oder inhalativer Gabe 0.01 bis 10 mg/kg Körpergewicht, vorzugsweise 0.1 bis 10 mg/kg Körpergewicht, jeweils 1 bis 3 x täglich.

Sofern die Behandlung mit CGRP-Antagonisten oder/und CGRP-Release-Hemmern in Ergänzung zu einer üblichen Hormonsubstitution erfolgt, empfiehlt sich eine Verringerung der vorstehend angegebenen Dosierungen, wobei die Dosierung dann 1/5 der vorstehend angegebenen Untergrenzen bis zu 1/1 der vorstehend angegebenen Obergrenzen betragen kann.

Die erfindungsgemäß hergestellten Verbindungen können entweder alleine oder gegebenenfalls in Kombination mit anderen Wirksubstanzen zur Behandlung von Migräne intravenös, subkutan, intramuskulär, intrarektal, intranasal, durch Inhalation, transdermal oder oral erfolgen, wobei zur Inhalation insbesondere Aerosolformulierungen geeignet sind. Die Kombinationen können entweder simultan oder sequentiell verabreicht werden.

Als Kombinationspartner denkbare Wirkstoffklassen sind z.B. Angiotensin-II Rezeptorantagonisten, α-Agonisten und α-Antagonisten, 5-HT_{1B/1D}-Agonisten, AMPA-Antagonisten, schwachen Analgetica, Antidepressiva, Antiemetika, Antikonvulsiva, Antimuscarinika, β-Blocker, Calcium-Antagonisten, Corticosteroide, Ergot-Alkaloiden, Histamin-H1-Rezeptorantagonisten, Neurokinin-Antagonisten, Neuroleptika, nichtsteroidale Antiphlogistika, NO-Synthase-Hemmer, Prokinetika, selektive Serotonin-Wiederaufnahme-Hemmer oder andere Antimigränemitteln, die zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragées, Kapseln, Pulver, Suspensionen, Lösungen, Dosieraerosole oder Zäpfchen eingearbeitet werden können.

Für die oben erwähnten Kombinationen kommen somit als weitere Wirksubstanzen beispielsweise die nicht-steroidalen Antiphlogistika Aceclofenac, Acemetacin, Acetylsalicylsäure, Azathioprin, Diclofenac, Diflunisal, Fenbufen, Fenoprofen, Flurbiprofen, Ibuprofen, Indometacin, Ketoprofen, Leflunomid, Lornoxicam, Mefenaminsäure, Naproxen, Phenylbutazon, Piroxicam, Sulfasalazin, Tenoxicam, Zomepirac oder deren physiologisch verträgliche Salze sowie Meloxicam und andere selektive COX2-Inhibitoren, wie beispielsweise Rofecoxib und Celecoxib, in Betracht.

Weiterhin können z.B. Candesartan, Eprosartan, Irbesartan, Losartan, Olmesartan, Tasosartan, Telmisartan, Valsartan, Duloxetin, Ergotamin, Dihydroergotamin, Metoclopramid, Domperidon, Diphenhydramin, Cyclizin, Promethazin, Chlorpromazin, Vigabatrin, Timolol, Isomethepten, Pizotifen, Botox, Gabapentin, Topiramat, Riboflavin, Montelukast, Lisinopril, Prochlorperazin, Dexamethason, Flunarizin, Dextropropoxyphen, Meperidin, Metoprolol, Propranolol, Nadolol, Atenolol, Clonidin, Indoramin, Carbamazepin, Phenytoin, Valproat, Amitryptilin, Lidocain oder Diltiazem und andere 5-HT_{1B/1D}-Agonisten wie z.B. Almotriptan, Avitriptan, Donitriptan, Eletriptan, Frovatriptan, Naratriptan, Rizatriptan, Sumatriptan und Zolmitriptan sowie deren physiologisch verträgliche Salze verwendet werden.

Die Dosis für diese Wirksubstanzen beträgt hierbei zweckmäßigerweise 1/5 der üblicherweise empfohlenen niedrigsten Dosierung bis zu 1/1 der normalerweise empfohlenen Dosierung, also beispielsweise 20 bis 100 mg Sumatriptan.

Ein weiterer Gegenstand der Erfindung ist die Verwendung der erfindungsgemäßen Verbindungen als wertvolle Hilfsmittel zur Erzeugung und Reinigung (Affinitätschromatographie) von Antikörpern sowie, nach geeigneter radioaktiver Markierung, beispielsweise durch Tritiierung geeigneter Vorstufen, beispielsweise durch katalytische Hydrierung mit Trithium oder Ersatz von Halogenatomen durch Tritium, in RIA- und ELISA-Assays und als diagnostische bzw. analytische Hilfsmittel in der Neurotransmitter-Forschung.

### Experimenteller Teil

Für die hergestellten Verbindungen liegen in der Regel IR-, ¹H-NMR und/oder Massenspektren vor. Wenn nicht anders angegeben, werden R_{f}-Werte unter Verwendung von DC-Fertigplatten Kieselgel 60 F254 (E. Merck, Darmstadt, Artikel-Nr. 1.05714) ohne Kammersättigung bestimmt. Die unter der Bezeichnung Alox ermittelten R_{f}-Werte werden unter Verwendung von DC-Fertigplatten Aluminiumoxid 60 F254 (E. Merck, Darmstadt, Artikel-Nr. 1.05713) ohne Kammersättigung bestimmt. Die bei den Fliessmitteln angegebenen Verhältnisse beziehen sich auf Volumeneinheiten der jeweiligen Lösungsmittel. Die angegebenen Volumeneinheiten bei NH₃ beziehen sich auf eine konzentrierte Lösung von NH₃ in Wasser.

Soweit nicht anders vermerkt sind die bei den Aufarbeitungen der Reaktionslösungen verwendeten Säure-, Basen- und Salzlösungen wässrige Systeme der angebenen Konzentrationen.

Zu chromatographischen Reinigungen wird Kieselgel der Firma Millipore (MATREX*^{™}*, 35-70 µm) verwendet. Zu chromatographischen Reinigungen wird Alox (E. Merck, Darmstadt, Aluminiumoxid 90 standardisiert, 63-200 µm, Artikel-Nr: 1.01097.9050) verwendet.

Die angegebenen HPLC-Daten werden unter nachstehend angeführten Parametern gemessen:
Analytische Säule: Zorbax-Säule (Agilent Technologies), SB (Stable Bond) - C18; 3.5 µm; 4.6 x 75 mm; Säulentemperatur: 30°C; Fluss: 0.8, mL / min; Injektionsvolumen: 5 µL; Detektion bei 254 nm

### Methode A:

| Zeit | Volumenprozent Wasser | Volumenprozent Acetonitril |
|---|---|---|
| (min) | (mit 0.1% Ameisensäure) | (mit 0.1% Ameisensäure) |
| 0 | 90 | 10 |
| 9 | 10 | 90 |
| 10 | 10 | 90 |
| 11 | 90 | 10 |

Bei präparativen HPLC-Reinigungen werden in der Regel die gleichen Gradienten verwendet, die bei der Erhebung der analytischen HPLC-Daten benutzt wurden.

Die Sammlung der Produkte erfolgt massengesteuert, die Produkt enthaltenden Fraktionen werden vereinigt und gefriergetrocknet.

Falls nähere Angaben zur Konfiguration fehlen, bleibt offen, ob es sich um reine Enantiomere handelt oder ob partielle oder gar völlige Racemisierung eingetreten ist.

In den Versuchsbeschreibungen werden die folgenden Abkürzungen verwendet:
- abs.: absolutiert
- Boc: *tert*.-Butoxycarbonyl
- CDI: *N*,*N*'-Carbonyldiimidazol
- CDT: 1,1'-Carbonyldi-(1,2,4-triazol)
- Cyc: Cyclohexan
- DCM: Dichlormethan
- DMAP: 4-Dimethylaminopyridin
- DMF: N,N-Dimethylformamid
- EtOAc: Essigsäureethylester
- EtOH: Ethanol
- halbkonz.: halbkonzentriert
- HCl: Salzsäure
- HOAc: Essigsäure
- HOBt: 1-Hydroxybenzotriazol-hydrat
- i. vac.: in vacuo (im Vakuum)
- KOH: Kaliumhydroxid
- konz.: konzentriert
- MeOH: Methanol
- NaCl: Natriumchlorid
- NaOH: Natriumhydroxid
- NMP: N-Methylpyrrolidon
- org.: organisch
- PE: Petrolether
- RT: Raumtemperatur
- TBME: *tert*.-Butyl-methylether
- TBTU: 2-(1*H*-Benzotriazol-1-yl)-1,1,3,3-tetramethyluronium-Tetrafluorborat
- TFA: Trifluoressigsäure
- THF: Tetrahydrofuran

### Beispiel 1

4-(2-Oxo-1,2,4,5-tetrahydro-benzo[*d*][1,3]diazepin-3-yl)-piperidin-1-carbonsäure-(*R*)-1-(4-amino-3-chlor-5-trifluormethyl-benzyl)-2-oxo-2-[4-(2-piperidin-1-yl-ethyl)-piperidin-1-yl]-ethylester

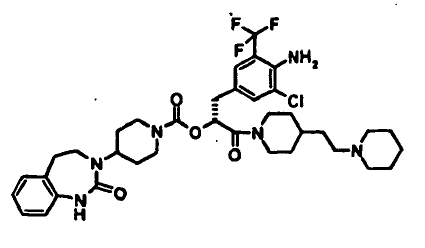

### (1a) (E)-2-Acetylamino-3-(4-amino-3-chlor-5-trifluormethyl-phenyl)-acrylsäure

Eine Mischung aus 50.0 g (224 mmol) 4-Amino-3-chlor-5-trifluormethyl-benzaldehyd, 39.3 g (335 mmol) N-Acetylglycin, 27.5 g (335 mmol) Natriumacetat und 200 ml Acetanhydrid wurde 2 Stunden in einem Ölbad bei 128°C Ölbadtemperatur gerührt. Nach Abkühlen auf 90°C Ölbadtemperatur wurden 100 ml Wasser zugetropft und die entstehende Suspension auf eine Mischung aus 1000 ml Wasser und 500 ml Toluol gegeben. Der ausgefallene Niederschlag wurde abgesaugt, mit 300 ml Toluol und 500 ml Wasser gewaschen und im Umlufttrockenschrank bei 60°C über Nacht getrocknet.

| | |
|---|---|
| Ausbeute: | 51.0 g (71% der Theorie) |
| ESI-MS: | (M+H)⁺ = 323 / 325 (CI) |

### (1b) (E)-3-(4-Amino-3-chlor-5-trifluormethyl-phenyl)-2-hvdroxy-acrylsäure

51.0 g (158 mmol) (*E*)-2-Acetylamino-3-(4-amino-3-chlor-5-trifluormethyl-phenyl)-acrylsäure, gelöst in 408 ml NMP, wurden mit 612 ml wässeriger 4-molarer Salzsäurelösung versetzt und 3 Stunden bei einer Badtemperatur von 130°C gerührt. Das Reaktionsgemisch wurde abgekühlt und unter Rühren auf 2000 ml Wasser gegossen. Der entstandene Niederschlag wurde abgesaugt, mit 400 ml Wasser gewaschen, bei 60°C über Nacht getrocknet und aus 1000 ml siedendem Toluol umkristallisiert.

| | |
|---|---|
| Ausbeute: | 24.2 g (54% der Theorie) |
| MS: | (M)⁺= 281 / 283 (CI) |

### (1c) (R)-3-(4-Amino-3-chlor-5-trifluormethyl-phenyl)-2-hvdroxy-propionsäure

Zu einer auf -20°C gekühlten Mischung aus 24.5 g (86.9 mmol) (*E*)-3-(4-Amino-3-chlor-5-trifluormethyl-phenyl)-2-hydroxy-acrylsäure, 12.1 ml (86.9 mmol) Triethylamin und 98 ml THF wurde unter Stickstoff Schutzgas 33,5 g (104.3 mmol) (-)-DIP-chlorid gelöst in 195 ml THF zugegeben. Das Reaktionsgemisch wurde 1.5 Stunden bei -20°C gerührt, auf Raumtemperatur gebracht und unter vermindertem Druck eingeengt. Der Rückstand wurde mit 200 ml wässeriger 1-molarer Natronlauge und 150 ml TBME versetzt und gut verrührt. Die wässerige Phase wurde abgetrennt, unter Rühren mit 2-molarer Salzsäurelösung angesäuert und zweimal mit je 250 ml TBME extrahiert. Die vereinigten organischen Phasen wurden über Aktivkohle filtriert und unter vermindertem Druck eingeengt. Der Rückstand wurde mit 500 ml Wasser zum Sieden erhitzt und die heiße Lösung über Celite klar filtriert. Der bei Raumtemperatur entstehende Niederschlag wurde abgesaugt und im Umluftrockenschrank bei 65°C getrocknet.

| | |
|---|---|
| Ausbeute: | 14.3 g (58% der Theorie) |
| MS: | (M+H)⁺ = 284 / 286 (CI) |

### (1d) (R)-3-(4-Amino-3-chlor-5-trifluormethyl-phenyl)-2-hydroxy-propionsäure-ethylester

Eine Mischung aus 14.3 g (50.0 mmol) (*R*)-3-(4-Amino-3-chlor-5-trifluormethylphenyl)-2-hydroxy-propionsäure und 100 ml Ethanol wurde mit 100 ml einer ca. 12-molaren ethanolischen Salzsäurelösung versetzt und über Nacht gerührt. Das Reaktionsgemisch wurde unter vermindertem Druck eingeengt.

| | |
|---|---|
| Ausbeute: | 15.7 g (100% der Theorie) |
| MS: | (M+H)⁺ = 312 / 314 (CI) |

### (1e) 4-(2-Oxo-1,2,4,5-tetrahydro-benzo[d][1,3]diazepin-3-yl)-picieridin-1-carbonsäure-(R)-2-(4-amino-3-chlor-5-trifluormethyl-phenyl)-1-ethoxycarbonyl-ethylester

Zu der Mischung aus 3.1 g (25.6 mmol) DMAP und 70 ml Pyridin wurden unter Stickstoff Schutzgas 5.2 g (25.6 mmol) Chlorameisensäure-4-nitrophenylester zugegeben und 1.5 Stunden bei Raumtemperatur gerührt. Anschließend wurden 8.0 g (25.7 mmol) (*R*)-3-(4-Amino-3-chlor-5-trifluormethyl-phenyl)-2-hydroxy-propionsäureethylester, gelöst in 30 ml Pyridin, langsam bei Raumtemperatur zugetropft, das Reaktionsgemisch 2 Stunden bei Raumtemperatur nachgerührt, 6.3 g (25.6 mmol) 3-Piperidin-4-yl-1,3,4,5-tetrahydro-1,3-benzdiazepin-2-on als Festsubstanz zugegeben und über Nacht bei Raumtemperatur gerührt. Das Reaktionsgemisch wurde unter vermindertem Druck eingeengt und zwischen 200 ml Essigester und 200 ml wässriger 10%-iger Zitronensäurelösung verteilt. Die organische Phase wurde zweimal mit je 200 ml 10%-iger Zitronensäurelösung und fünfmal mit je 150 ml 15%-iger wässriger Kaliumcarbonatlösung gewaschen, über Natriumsulfat getrocknet und unter vermindertem Druck eingeengt. Der Rückstand wurde säulenchromatographisch gereinigt.

| | |
|---|---|
| Ausbeute: | 5.0 g (33% der Theorie) |
| MS: | (M+H)⁺ = 583 / 585 (CI) |

### (1f) 4-(2-Oxo-1,2,4,5-tetrahydro-benzof[d][1,3]diazepin-3-yl)-piperidin-1-carbonsäure-(R)-2-(4-amino-3-chlor-5-trifluormethyl-Phenyl)-1-carboxyethylester

Zu der Mischung aus 13.0 g (22.3 mmol) 4-(2-Oxo-1,2,4,5-tetrahydro-benzo[*d*]-[1,3]diazepin-3-yl)-piperidin-1-carbonsäure-(*R*)-2-(4-amino-3-chlor-5-trifluormethylphenyl)-1-ethoxycarbonyl-ethylester und 120 ml THF wurde eine Lösung von 804 mg (33.5 mmol) Lithiumhydroxid gelöst in 80 ml Wasser zugetropft. Das Gemisch wurde 3 Stunden bei Raumtemperatur gerührt, unter vermindertem Druck von THF befreit, mit 150 ml Wasser versetzt, und durch Zugabe von wässeriger 4-molarer Salzsäurelösung angesäuert. Danach wurde die wässerige Phase mit 300 ml Essigester extrahiert, die organische Phase getrocknet und unter vermindertem Druck eingeengt. Der Rückstand wurde aus 50 ml Isopropanol umkristallisiert.
Ausbeute: 5.3 g (43% der Theorie)

### (1g) 4-(2-Oxo-1,2,4,5-tetrahydro-benzo[d][1,3]diazepin-3-yl)-piperidin-1-carbonsäure-(R)-1-(4-amino-3-chlor-5-trifluormethyl-benzyl)-2-oxo-2-[4-(2-piperidin-1-yl-ethyl)-piperidin-1-yl]-ethylester

Eine Mischung aus 100 mg (0.18 mmol) 4-(2-Oxo-1,2,4,5-tetrahydro-benzo[*d*]-[1,3]diazepin-3-yl)-piperidin-1-carbonsäure-(*R*)-2-(4-amino-3-chlor-5-trifluormethylphenyl)-1-carboxyethylester, 35.3 mg (0.18 mmol) 4-(2-piperidin-1-yl-ethyl)-piperidin, 64.2 mg (0.20 mmol) TBTU, 0.028 ml (0.20 mmol) Triethylamin und 2.0 ml DMF wurde 12 Stunden bei Raumtemperatur gerührt. Das Reaktionsgemisch wurde säulenchromatographisch aufgereinigt.

| | |
|---|---|
| Ausbeute: | 84 mg (64% der Theorie) |
| MS: | (M+H)⁺ = 733 / 735 (CI) |
| Retentionszeit HPLC: | 6.5 min (Methode A) |

Analog wurden folgende Verbindungen aus jeweils 100 mg 4-(2-Oxo-1,2,4,5-tetrahydro-benzo[*d*][1,3]diazepin-3-yl)-piperidin-1-carbonsäure-(*R*)-2-(4-amino-3-chlor-5-trifluormethyl-phenyl)-1-carboxy-ethylester und der entsprechenden Menge an Amin hergestellt:

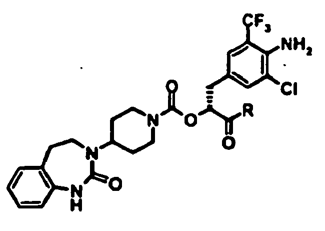

| **Beispiel** | **R** | **Ausbeute** | **Massen-** | **Retentionszeit** |
|---|---|---|---|---|
| | | **(%)** | **spektrum** | **HPLC** |
| | | | | **(Methode)** |
| 1.1 | 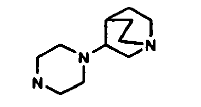 | 15 | 732/734 | 6.0 min |
| | | | [M+H]⁺ | (A) |
| 4-(2-Oxo-1,2,4,5-tetrahydro-benzo[*d*][1,3]diazepin-3-yl)-piperidin-1-carbonsäure-(*R*)-1-(4-amino-3-chlor-5-trifluormethyl-benzyl)-2-[4-(1-aza-bicyclo[2.2.2]oct-3-yl)-piperazin-1-yl]-2-oxo-ethylester | | | | |
| 1.2 | 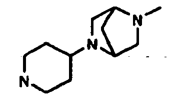 | 74 | 732/734 | 5.5 min |
| | | | [M+H]⁺ | (A) |
| 4-(2-Oxo-1,2,4,5-tetrahydro-benzo[*d*][1,3]diazepin-3-yl)-piperidin-1-carbonsäure-(*R*)-1-(4-amino-3-chlor-5-trifluormethyl-benzyl)-2-[4-(5-methyl-2,5-diaza-bicyclo-[2.2.1]hept-2-yl)-piperidin-1-yl]-2-oxo-ethylester | | | | |
| 1.3 | 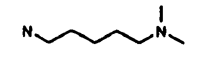 | 92 | 667/669 | 5.9 min |
| | | | [M+H]⁺ | (A) |
| 4-(2-Oxo-1,2,4,5-tetrahydro-benzo[*d*][1,3]diazepin-3-yl)-piperidin-1-carbonsäure-(*R*)-2-(4-amino-3-chlor-5-trifluormethyl-phenyl)-1-(5-dimethylamino-pentylcarba-moyl)-ethylester | | | | |
| 1.4 | 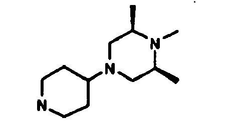 | 65 | 748/750 | 5.5 min |
| | | | [M+H]⁺ | (A) |
| 4-(2-Oxo-1,2,4,5-tetrahydro-benzo[*d*][1,3]diazepin-3-yl)-piperidin-1-carbonsäure-(*R*)-1-(4-amino-3-chlor-5-trifluormethyl-benzyl)-2-oxo-2-[4-((3*R*,5*S*)-3,4, 5-trimethyl-piperazin-1-yl)-piperidin-1-yl]-ethylester | | | | |
| 1.5 | 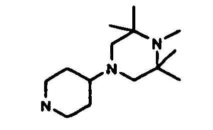 | 61 | 776/778 | 6.3 min |
| | | | [M+H]⁺ | (A) |
| 4-(2-Oxo-1,2,4,5-tetrahydro-benzo[*d*][1,3]diazepin-3-yl)-piperidin-1-carbonsäure-(*R*)-1-(4-amino-3-chlor-5-trifluormethyl-benzyl)-2-oxo-2-[4-(3,3,4,5,5-pentamethyl-piperazin-1-yl)-piperidin-1-yl]-ethylester | | | | |
| 1.6 | 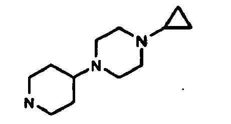 | 54 | 746/748 | 5.3 min |
| | | | [M+H]⁺ | (A) |
| 4-(2-Oxo-1,2,4,5-tetrahydro-benzo[*d*][1,3]diazepin-3-yl)-piperidin-1-carbonsäure-(*R*)-1-(4-amino-3-chlor-5-trifluormethyl-benzyl)-2-[4-(4-cyclopropyl-piperazin-1-yl)-piperidin-1-yl]-2-oxo-ethylester | | | | |
| 1.7 | 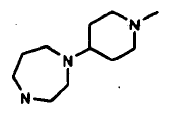 | 82 | 734/736 | 5.3 min |
| | | | [M⁺H]⁺ | (A) |
| 4-(2-Oxo-1,2,4,5-tetrahydro-benzo[*d*][1,3]diazepin-3-yl)-piperidin-1-carbonsäure-(*R*)-1-(4-amino-3-chlor-5-trifluormethyl-benzyl)-2-[4-(1-methyl-piperidin-4-yl)-[1,4]-diazepan-1-yl]-2-oxo-ethylester | | | | |
| 1.8 | 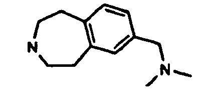 | 61 | 741/743 | 6.7 min |
| | | | [M+H]⁺ | (A) |
| 4-(2-Oxo-1,2,4,5-tetrahydro-benzo[*d*][1,3]diazepin-3-yl)-piperidin-1-carbonsäure-(R)-1-(4-amino-3-chlor-5-trifluormethyl-benzyl)-2-(7-dimethylaminomethyl-1,2,4,5-tetrahydro-benzo[*d*]azepin-3-yl)-2-oxo-ethylester | | | | |
| 1.9 | 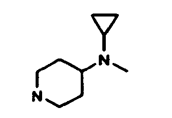 | 45 | 691/693 | 6.3 min |
| | | | [M+H]⁺ | (A) |
| 4-(2-Oxo-1,2,4,5-tetrahydro-benzo[*d*][1,3]diazepin-3-yl)-piperidin-1-carbonsäure- | | | | |
| (*R*)-1-(4-amino-3-chlor-5-trifluormethyl-benzyl)-2-[4-(cyclopropyl-methyl-amino)-piperidin-1-yl]-2-oxo-ethylester | | | | |
| 1.10 | 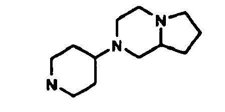 | 62 | 746/748 | 5.9 min |
| | | | [M+H]⁺ | (A) |
| 4-(2-Oxo-1,2,4,5-tetrahydro-benzo[*d*][1,3]diazepin-3-yl)-piperidin-1-carbonsäure-(*R*)-1-(4-amino-3-chlor-5-trifluomnethyl-benzyl)-2-[4-(hexahydro-pyrrolo[1,2-a]-pyrazin-2-yl)-piperidin-1-yl]-2-oxo-ethylester | | | | |
| 1.11 | 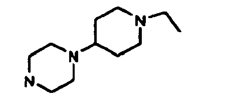 | 37 | 734/736 | 5.3 min |
| | | | [M+H]⁺ | (A) |
| 4-(2-Oxo-1,2,4,5-tetrahydro-benzo[*d*][1,3]diazepin-3-yl)-piperidin-1-carbonsäure-(*R*)-1-(4-amino-3-chlor-5-trifluormethyl-benzyl)-2-[4-(1-ethyl-piperidin-4-yl)-piperazin-1-yl]-2-oxo-ethylester | | | | |
| 1.12 | 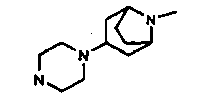 | 22 | 746/748 | 5.4 min |
| | | | [M+H]⁺ | (A) |
| 4-(2-Oxo-1,2,4,5-tetrahydro-benzo[*d*][1,3]diazepin-3-yl)-piperidin-1-carbonsäure-(*R*)-1-(4-amino-3-chlor-5-trifluormethyl-benzyl)-2-[4-(8-methyl-8-aza-bicyclo[3.2.1]-oct-3-yl)-piperazin-1-yl]-2-oxo-ethylester | | | | |
| 1.13 | 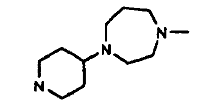 | 16 | 734/736 | 5.4 min |
| | | | [M+H]⁺ | (A) |
| 4-(2-Oxo-1,2,4,5-tetrahydro-benzo[*d*][1,3]diazepin-3-yl)-piperidin-1-carbonsäure-(*R*)-1-(4-amino-3-chlor-5-trifluormethyl-benzyl)-2-[4-(4-methyl-[1,4]diazepan-1-yl)-piperidin-1-yl]-2-oxo-ethylester | | | | |
| 1.14 | 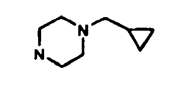 | 88 | 677/679 | 5.4 min |
| | | | [M+H]⁺ | (A) |
| 4-(2-Oxo-1,2,4,5-tetrahydro-benzo[*d*][1,3]diazepin-3-yl)-piperidin-1-carbonsäure- | | | | |
| (*R*)-1-(4-amino-3-chlor-5-trifluormethyl-benzyl)-2-(4-cyclopropylmethyl-piperazin-1-yl)-2-oxo-ethylester | | | | |
| 1.15 | 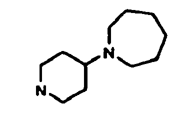 | 53 | 719/721 | 6.5 min |
| | | | [M+H]⁺ | . (A) |
| 4-(2-Oxo-1,2,4,5-tetrahydro-benzo[*d*][1,3]diazepin-3-yl)-piperidin-1-carbonsäure-(*R*)-1-(4-amino-3-chlor-5-trifluormethyl-benzyl)-2-(4-azepan-1-yl-piperidin-1-yl)-2-oxo-ethylester | | | | |
| 1.16 | 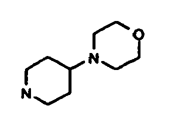 | 68 | 707/709 | 6.0 min |
| | | | [M+H]⁺ | (A) |
| 4-(2-Oxo-1,2,4,5-tetrahydro-benzo[*d*][1,3]diazepin-3-yl)-piperidin-1-carbonsäure-(*R*)-1-(4-amino-3-chlor-5-trifluormethyl-benzyl)-2-(4-morpholin-4-yl-piperidin-1-yl)-2-oxo-ethylester | | | | |
| 1.17 | 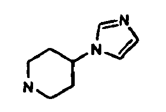 | 33 | 688/690 | 6.2 min |
| | | | [M+H]⁺ | (A) |
| 4-(2-Oxo-1,2,4,5-tetrahydro-benzo[*d*][1,3]diazepin-3-yl)-piperidin-1-carbonsäure-(*R*)-1-(4-amino-3-chlor-5-trifluormethyl-benzyl)-2-(4-imidazol-1-yl-piperidin-1-yl)-2-oxo-ethylester | | | | |

### Beispiel 3

4-(2-Oxo-1,2,4,5-tetrahydro-benzo[*d*][1,3]diazepin-3-yl)-piperidin-1-carbonsäure-(*R*)-1-(4-chlor-3-trifluormethyl-benzyl)-2-[4-(4-methyl-piperazin-1-yl)-piperidin-1-yl]-2-oxo-ethylester

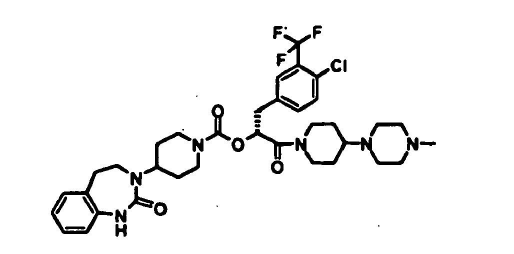

### (3a) (E)-2-Acetylamino-3-(4-chlor-3-trifluormethyl-phenyl)-acrylsäure

Hergestellt analog Beispiel 1a.

| | |
|---|---|
| Ausbeute: | 75% der Theorie |
| ESI-MS: | (M+H)⁺ = 308 / 310 (CI) |

### (3b) (E)-3-(4-Chlor-3-trifluormethyl-phenyl)-2-hydroxy-acrylsäure

Hergestellt analog Beispiel 1b.

| | |
|---|---|
| Ausbeute: | 55% der Theorie |
| MS: | (M-H)⁻ = 265 / 267 (CI) |

### (3c) (R)-3-(4-Chlor-3-trifluormethyl-phenyl)-2-hydroxy-propionsäure

Hergestellt analog Beispiel 1c.

| | |
|---|---|
| Ausbeute: | 64% der Theorie |
| ESI-MS: | (M-H)⁻ = 267 /269 (Cl) |

### (3d) (R)-3-(4-Chlor-3-trifluormethyl-phenyl)-2-hvdroxy-propionsäuremethylester

Hergestellt analog Beispiel 1d.

| | |
|---|---|
| Ausbeute: | 78% der Theorie |
| ESI-MS: | (M)⁺ = 282 /284 (CI) |

### (3e) 4-(2-Oxo-1,2,4,5-tetrahydro-benzo[d][1,3]diazepin-3-yl)-piperidin-1-carbonsäure-(R)-2-(4-chlor-3-trifluormethyl-phenyl)-1-methoxycarbonyl-ethylester

Hergestellt analog Beispiel 1 e.

| | |
|---|---|
| Ausbeute: | 22% der Theorie |
| ESI-MS: | (M+H)⁺ = 554 /556 (CI) |

### (3f) 4-(2-Oxo-1,2,4,5-tetrahvdro-benzo[d][1,3]diazepin-3-yl)-Diperidin-1-carbonsäure-(R)-2-(4-chlor-3-trifluormethyl-phenyl)-1-carboxyethylester

Hergestellt analog Beispiel 1f.

| | |
|---|---|
| Ausbeute: | 77% der Theorie |
| ESI-MS: | (M+H)⁺ = 540 /542 (CI) |

### (3g) 4-(2-Oxo-1,2,4,5-tetrahydro-benzo[d][1,3]diazepin-3-yl)-piperidin-1-carbonsäure-(R)-1-(4-chlor-3-trifluormethyl-benzyl)-2-[4-(4-methyl-piperazin-1-yl)-piperidin-1-yl]-2-oxo-ethylester

Hergestellt an analog Beispiel 1g.

| | |
|---|---|
| Ausbeute: | 40% der Theorie |
| ESI-MS: | (M+H)⁺ = 705 /707 (CI) |
| R_{f}: | 0.4 (Methylenchlorid/Cyclohexan/Methanol/Ammoniak = 70/15/15/2) |

### Beispiel 3.1

4-(2-Oxo-1,2,4,5-tetrahydro-benzo[*d*][1,3]diazepin-3₋yl)-piperidin-1-carbonsäure-(*R*)-1-(4-chlor-3-trifluormethyl-benzyl)-2-[4-(1-methyl-piperidin-4-yl)-piperazin-1-yl]-2-oxo-ethylester

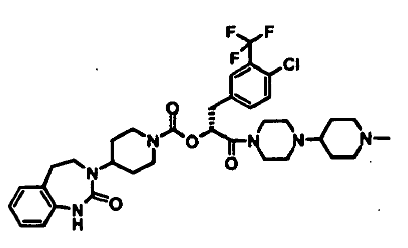

Hergestellt analog Beispiel 3g.

| | |
|---|---|
| Ausbeute: | 26% der Theorie |
| ESI-MS: | (M+H)⁺ = 705 /707 (CI) |
| R_{f}: | 0.4 (Methylenchlorid/Cyclohexan/Methanol/Ammoniak = 70/15/15/2) |

Die nachfolgenden Beispiele beschreiben die Herstellung pharmazeutischer Anwendungsformen, die als Wirkstoff eine beliebige Verbindung der allgemeinen Formel (I) enthalten:

### Beispiel I:

Kapseln zur Pulverinhalation mit 1 mg Wirkstoff
Zusammensetzung:
1 Kapsel zur Pulverinhalation enthält:

| | |
|---|---|
| Wirkstoff . | 1.0 mg |
| Milchzucker | 20.0 mg |
| Hartgelatinekapseln | 50.0 mg |
| | 71.0 mg |

Herstellungsverfahren:
Der Wirkstoff wird auf die für Inhalativa erforderliche Korngröße gemahlen. Der gemahlene Wirkstoff wird mit dem Milchzucker homogen gemischt. Die Mischung wird in Hartgelatinekapseln abgefüllt.

### Beispiel II:

Inhalationslösung für Respimat^{®} mit 1 mg Wirkstoff
Zusammensetzung:
1 Hub enthält:

| | |
|---|---|
| Wirkstoff | 1.0 mg |
| Benzalkoniumchlorid | 0.002 mg |
| Dinatriumedetat | 0.0075 mg |
| Wasser gereinigt ad | 15.0 µl |

Herstellungsverfahren:
Der Wirkstoff und Benzalkoniumchlorid werden in Wasser gelöst und in Respimat^{®}-Kartuschen abgefüllt.

### Beispiel III:

Inhalationslösung für Vernebler mit 1 mg Wirkstoff
Zusammensetzung:
1 Fläschchen enthält:

| | |
|---|---|
| Wirkstoff | 0.1 g |
| Natriumchlorid | 0.18 g |
| Benzalkoniumchlorid | 0.002 g |
| Wasser gereinigt ad | 20.0 ml |

Herstellungsverfahren:
Wirkstoff, Natriumchlorid und Benzalkoniumchlorid werden in Wasser gelöst.

### Beispiel IV:

Treibgas-Dosieraerosol mit 1 mg Wirkstoff
Zusammensetzung:
1 Hub enthält:

| | |
|---|---|
| Wirkstoff | 1.0 mg |
| Lecithin | 0.1 % |
| Treibgas ad | 50.0 µl |

Herstellungsverfahren:
Der mikronisierte Wirkstoff wird in dem Gemisch aus Lecithin und Treibgas homogen suspendiert. Die Suspension wird in einen Druckbehälter mit Dosierventil abgefüllt.

### Beispiel V:

Nasalspray mit 1 mg Wirkstoff
Zusammensetzung:

| | |
|---|---|
| Wirkstoff | 1.0 mg |
| Natriumchlorid | 0.9 mg |
| Benzalkoniumchlorid | 0.025 mg |
| Dinatriumedetat | 0.05 mg |
| Wasser gereinigt ad | 0.1 ml |

Herstellungsverfahren:
Der Wirkstoff und die Hilfsstoffe werden in Wasser gelöst und in ein entsprechendes Behältnis abgefüllt.

### Beispiel VI:

Injektionslösung mit 5 mg Wirksubstanz pro 5 ml
Zusammensetzung:

| | |
|---|---|
| Wirksubstanz | 5 mg |
| Glucose | 250 mg |
| Human-Serum-Albumin | 10 mg |
| Glykofurol | 250 mg |
| Wasser für Injektionszwecke ad | 5 ml |

Herstellung:
Glykofurol und Glucose in Wasser für Injektionszwecke auflösen (Wfl); Human-Serum-Albumin zugeben; Wirkstoff unter Erwärmen auflösen; mit Wfl auf Ansatzvolumen auffüllen; unter Stickstoff-Begasung in Ampullen abfüllen.

### Beispiel VII:

Injektionslösung mit 100 mg Wirksubstanz pro 20 ml
Zusammensetzung:

| | |
|---|---|
| Wirksubstanz | 100 mg |
| Monokaliumdihydrogen- | |
| phosphat = KH₂PO₄ | 12 mg |
| Dinatriumhydrogen | |
| phosphat = Na₂HPO₄·2H₂O | 2 mg |
| Natriumchlorid | 180 mg |
| Human-Serum-Albumin | 50 mg |
| Polysorbat 80 | 20 mg |
| Wasser für Injektionszwecke ad | 20 ml |

Herstellung:
Polysorbat 80, Natriumchlorid, Monokaliumdihydrogenphosphat und Dinatriumhydrogenphosphat in Wasser für Injektionszwecke (Wfl) auflösen; Human-Serum-Albumin zugeben; Wirkstoff unter Erwärmen auflösen; mit Wfl auf Ansatzvolumen auffüllen; in Ampullen abfüllen.

### Beispiel VIII:

Lyophilisat mit 10 mg Wirksubstanz
Zusammensetzung:

| | |
|---|---|
| Wirksubstanz | 10 mg |
| Mannit | 300 mg |
| Human-Serum-Albumin | 20 mg |

Herstellung:
Mannit in Wasser für Injektionszwecke (Wfl) auflösen; Human-Serum-Albumin zugeben; Wirkstoff unter Erwärmen auflösen; mit Wfl auf Ansatzvolumen auffüllen; in Vials abfüllen; gefriertrocknen.

Lösungsmittel für Lyophilisat:

| | |
|---|---|
| Polysorbat 80 = Tween 80 | 20 mg |
| Mannit | 200 mg |
| Wasser für Injektionszwecke ad | 10 ml |

Herstellung:
Polysorbat 80 und Mannit in Wasser für Injektionszwecke (Wfl) auflösen; in Ampullen abfüllen.

### Beispiel IX:

Tabletten mit 20 mg Wirksubstanz
Zusammensetzung:

| | |
|---|---|
| Wirksubstanz | 20 mg |
| Lactose | 120 mg |
| Maisstärke | 40 mg |
| Magnesiumstearat | 2 mg |
| Povidon K 25 | 18 mg |

Herstellung:
Wirksubstanz, Lactose und Maisstärke homogen mischen; mit einer wässerigen Lösung von Povidon granulieren; mit Magnesiumstearat mischen; auf einer Tablettenpresse abpressen; Tablettengewicht 200 mg.

### Beispiel X:

Kapseln mit 20 mg Wirksubstanz
Zusammensetzung:

| | |
|---|---|
| Wirksubstanz | 20 mg |
| Maisstärke | 80 mg |
| Kieselsäure. hochdispers | 5 mg |
| Magnesiumstearat | 2.5 mg |

Herstellung:
Wirksubstanz, Maisstärke und Kieselsäure homogen mischen; mit Magnesiumstearat mischen; Mischung auf einer Kapselfüllmaschine in Hartgelatine-Kapseln Grösse 3 abfüllen.

### Beispiel XI:

Zäpfchen mit 50 mg Wirksubstanz
Zusammensetzung:

| | |
|---|---|
| Wirksubstanz | 50 mg |
| Hartfett (Adeps solidus) q.s. ad | 1700 mg |

Herstellung:
Hartfett bei ca. 38°C aufschmelzen; gemahlene Wirksubstanz im geschmolzenen Hartfett homogen dispergieren; nach Abkühlen auf ca. 35°C in vorgekühlte Formen ausgiessen.

### Beispiel XII:

Injektionslösung mit 10 mg Wirksubstanz pro 1 ml
Zusammensetzung:

| | |
|---|---|
| Wirksubstanz | 10 mg |
| Mannitol | 50 mg |
| Human-Serum-Albumin | 10 mg |
| Wasser für Injektionszwecke ad | 1 ml |

Herstellung:
Mannitol in Wasser für Injektionszwecke auflösen (Wfl); Human-Serum-Albumin zugeben; Wirkstoff unter Erwärmen auflösen; mit Wfl auf Ansatzvolumen auffüllen; unter Stickstoff-Begasung in Ampullen abfüllen.

## Patentansprüche

1. Einzelne CGRP-Antagonisten der allgemeinen Formel (I) 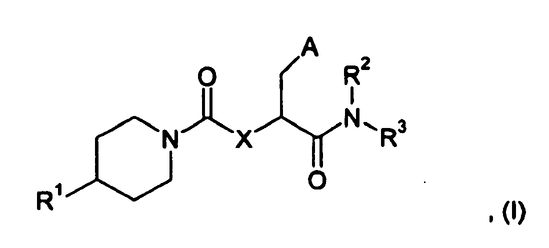 in der
A einen Rest der Formel 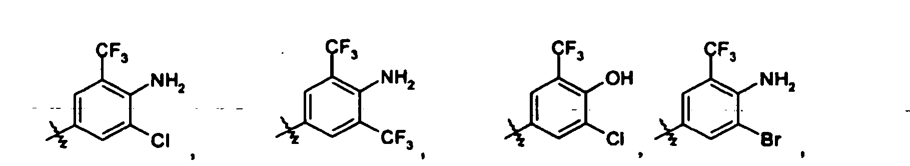 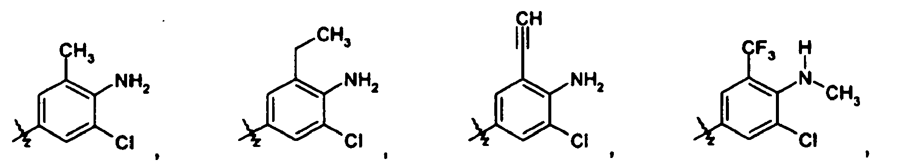 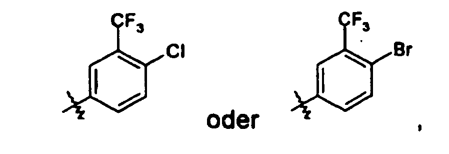 X ein Sauerstoffatom, eine Methylen- oder NH-Gruppe, R¹ einen Rest der Formel 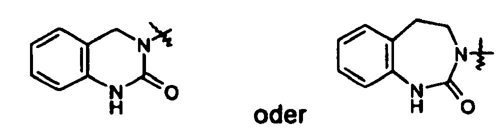 und
-NR²R³ einen Rest der Formel 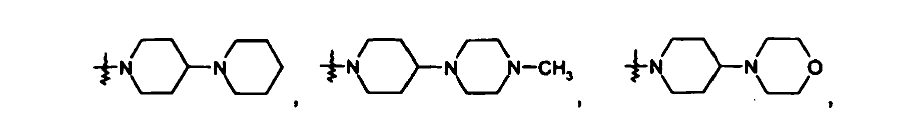 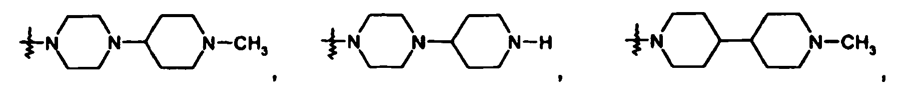 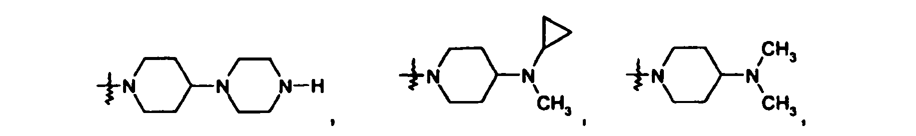 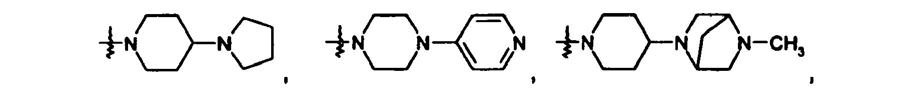 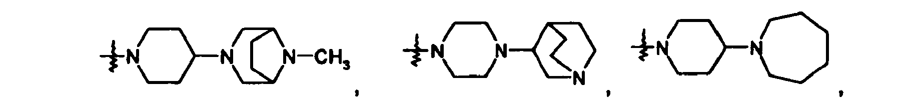 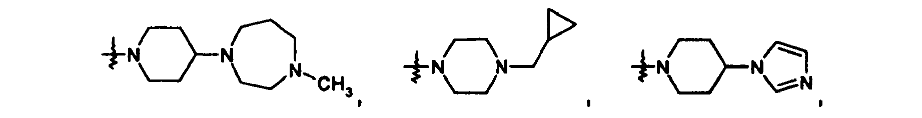 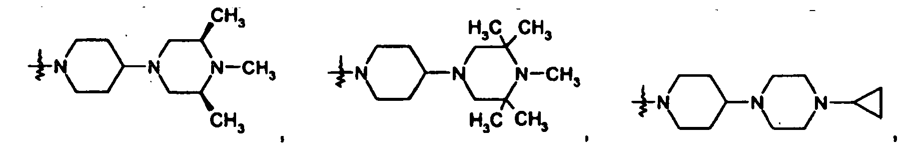 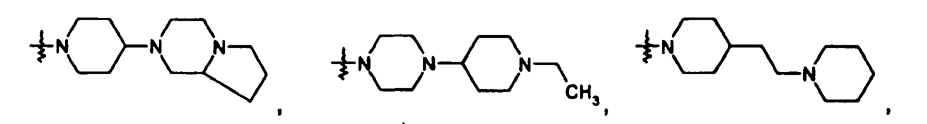 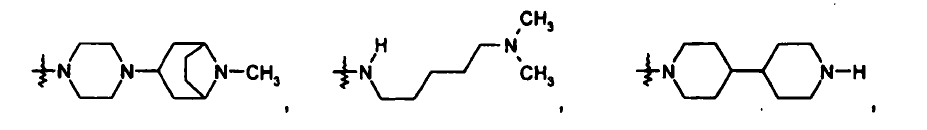 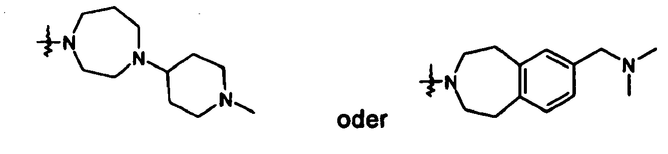 bedeutet, nämlich:
(1) 4-(2-Oxo-1,2,4,5-tetrahydro-benzo[*d*][1,3]diazepin-3-yl)-piperidin-1-carbonsäure-(*R*)-1-(4-amino-3-chlor-5-trifluormethyl-benzyl)-2-oxo-2-[4-(2-piperidin-1-yl-ethyl)-piperidin-1-yl]-ethylester,
(2) 4-(2-Oxo-1,2 4,5-tetrahydro-benzo[*d*][1,3]diazepin-3-yl)-pipeddin-1-carbonsäure-(*R*)-1-(4-amino-3-chlor-5-trifluomethyl-benzyl)-2-[4-(1-aza-bicyclo-[2.2.2]oct-3-yl)-piperazin-1-yl]-2-oxo-ethylester,
(3) 4-(2-Oxo-1,2,4,5-tetrahydro-benzo[*d*][1,3]diazepin-3-yl)-piperidin-1-carbonsäure-(*R*)-1-(4-amino-3-chlor-5-trifluormethyl-benzyl)-2-[4-(5-methyl-2,5-diazabicyclo[2.2.1]hept-2-yl)-piperidin-1-yl]-2-oxo-ethylester,
(4) 4-(2-Oxo-1,2,4,5-tetrahydro-benzo[*d*][1,3]diazepin-3-yl)-piperidin-1-carbonsäure-(*R*)-2-(4-amino-3-chlor-5-trifluormethyl-phenyl)-1-(5-dimethylamino-pentylcarbamoyl)-ethylester,
(5) 4-(2-Oxo-1,2,4,5-tetrahydro-benzo[*d*][1,3]diazepin-3-yl)-piperidin-1-carbonsäure-(*R*)-1-(4-amino-3-chlor-5-trifluormethyl-benzyl)-2-oxo-2-[4-((3*R*,5*S*)-3,4,5-trimethyl-piperazin-1-yl)-piperidin-1-yl]-ethylester,
(6) 4-(2-Oxo-1,2,4,5-tetrahydro-benzo[*d*][1,3]diazepin-3-yl)-piperidin-1-carbonsäure-(*R*)-1-(4-amino-3-chlor-5-trifluormethyl-benzyl)-2-oxo-2-[4-(3,3,4,5,5-pentamethyl-piperazin-1-yl)-piperidin-1-yl]-ethytester,
(7) 4-(2-Oxo-1,2,4,5-tetrahydro-benzo[*d*][1,3]diazepin-3-yl)-pipendin-1-carbonsäure-(*R*)-1-(4-amino-3-chlor-5-trifluormethyl-benzyl)-2-[4-(4-cyclopropyl-piperazin-1-yl)-piperidin-1-yl]-2-oxo-ethylester,
(8) 4-(2-Oxo-1,2,4,5-tetrahydro-benzo[*d*][1,3]diazepin-3-yl)-piperidin-1-carbonsäure-(*R*)-1-(4-amino-3-chlor-5-trifluormethyl-benzyl)-2-[4-(1-methyl-piperidin-4-yl)-[1,4]diazepan-1-yl]-2-oxo-ethylester,
(9) 4-(2-Oxo-1,2,4,5-tetrahydro-benzo[*d*][1,3]diazepin-3-yl)-piperidin-1-carbonsäure-(*R*)-1-(4-amino-3-chlor-5-trifluormethyl-benzyl)-2-(7-dimethylaminomethyl-1,2,4,5-tetrahydro-benzo[*d*]azepin-3-yl)-2-oxo-ethylester,
(10) 4-(2-Oxo-1,2,4,5-tetrahydro-benzo[*d*][1,3]diazepin-3-yl)-piperidin-1-carbonsäure-(*R*)-1-(4-amino-3-chlor-5-trifluormethyl-benzyl)-2-[4-(cyclopropyl-methylamino)-piperidin-1-yl]-2-oxo-ethylester,
(11) 4-(2-Oxo-1,2,4,5-tetrahydro-benzo[*d*][1,3]diazepin-3-yl)-piperidin-1-carbonsäure-(*R*)-1-(4-amino-3-chlor-5-trifluormethyl-benzyl)-2-[4-(hexahydro-pyrrolo-[1,2-a]pyrazin-2-yl)-piperidin-1-yl]-2-oxo-ethylester,
(12) 4-(2-Oxo-1,2,4,5-tetrahydro-benzo[*d*][1,3]diazepin-3-yl)-piperidin-1-carbonsäure-(*R*)-1-(4-amino-3-chlor-5-trifluormethyl-benryl)-2-[4-(1-ethyl-piperidin-4-yl)-piperazin-1-yl]-2-oxo-ethylester,
(13) 4-(2-Oxo-1,2,4,5-tetrahydro-benzo[*d*][1,3]diazepin-3-yl)-piperidin-1-carbonsäure-(*R*)-1-(4-amino-3-chlor-5-trifluormethyl-benzyl)-2-[4-(8-methyl-8-azabicyclo[3.2.1]oct-3-yl)-piperazin-1-yl]-2-oxo-ethylester,
(14) 4-(2-Oxo-1,2,4,5-tetrahydro-benzo[*d*][1,3]diazepin-3-yl)-piperidin-1-carbonsäure-(*R*)-1-(4-amino-3-chlor-5-trifluormethyl-benzyl)-2-[4-(4-methyl-[1,4]diazepan-1-yl)-piperidin-1-yl]-2-oxo-ethylester,
(15) 4-(2-Oxo-1,2,4,5-tetrahydro-benzo[*d*][1,3]diazepin-3-yl)-piperidin-1-carbonsäure-(*R*)-1-(4-amino-3-chlor-5-trifluormethyl-benzyl)-2-(4-cyclopropylmethyl-piperazin-1-yl)-2-oxo-ethylester,
(16) 4-(2-Oxo-1,2,4,5-tetrahydro-benzo[*d*][1,3]diazepin-3-yl)-piperidin-1-carbonsäure-(*R*)-1-(4-amino-3-chtor-5-trifluormethyl-benzyl)-2-(4-azepan-1-yl-piperidin-1-yl)-2-oxo-ethylester,
(17) 4-(2-Oxo-1,2,4,5-tetrahydro-benzo[*d*][1,3]diazepin-3-yl)-piperidin-1-carbonsäure-(*R*)-1-(4-amino-3-chlor-5-trifluomethyl-benzyl)-2-(4-morpholin-4-yl-piperidin-1-yl)-2-oxo-ethylester,
(18) 4-(2-Oxo-1,2,4,5-tetrahydro-benzo[*d*][1,3]diazepin-3-yl)-piperidin-1-carbonsäure-(*R*)-1-(4-amino-3-chlor-5-trifluormethyl-benzyl)-2-(4-imidazol-1-yl-piperidin-1-yl)-2-oxo-ethylester,
(19) 4-(2-Oxo-1,2,4,5-tetrahydro-benzo[*d*][1,3]diazepin-3-yl)-piperidin-1-carbonsäure-(*R*)-1-(4-chlor-3-trifluormethyl-benzyl)-2-[4-(4-methyl-piperazin-1-yl)-piperidin-1-yl]-2-oxo-ethylester,
(20) 4-(2-Oxo-1,2,4,5-tetrahydro-benzo[*d*][1,3]diazepin-3-yl)-piperidin-1-carbonsäure-(*R*)-1-(4-chlor-3-trifluormethyl-benzyl)-2-[4-(1-methyl-piperidin-4-yl)-piperazin-1-yl]-2-oxo-ethylester,
deren Tautomere, deren Diastereomere, deren Enantiomere, deren Hydrate, deren Gemische und deren Salze sowie die Hydrate der Salze.

2. Physiologisch verträgliche Salze der Verbindungen gemäß Anspruch 1 mit anorganischen oder organischen Säuren.

3. Arzneimittel, enthaltend eine Verbindung gemäß Anspruch 1 oder ein physiologisch verträgliches Salz gemäß Anspruch 2 neben gegebenenfalls einem oder mehreren inerten Trägerstoffen und/oder Verdünnungsmitteln.

4. Verwendung einer Verbindung nach mindestens einem der Ansprüche 1 bis 2 zur Herstellung eines Arzneimittels zur akuten und prophylaktischen Behandlung von Kopfschmerzen, insbesondere Migräne- bzw. Cluster-Kopfschmerz.

5. Verwendung einer Verbindung nach mindestens einem der Ansprüche 1 bis 2 zur Herstellung eines Arzneimittels zur Bekämpfung von nicht-insulinabhängigem Diabetes-mellitus (NIDDM).

6. Verwendung einer Verbindung nach mindestens einem der Ansprüche 1 bis 2 zur Herstellung eines Arzneimittels zur Behandlung von CRPS1 (complex regional pain syndrome), von cardiovaskulären Erkrankungen, von Morphintoleranz, von Chlostritiumtoxin-bedingten Durchfallerkrankungen, von Erkrankungen der Haut, insbesondere von thermischen und strahlungsbedingten Schäden inklusive Sonnenbrand, von entzündlichen Erkrankungen wie insbesondere entzündlicher Gelenkerkrankungen wie Arthritis, von neurogenen Entzündungen der oralen Mucosa, von entzündlichen Lungenerkrankungen, von allergischer Rhinitis, von Asthma, von Erkrankungen, die mit einer überschießenden Gefäßerweiterung und **dadurch** bedingter verringerter Gefäßdurchblutung, wie insbesondere Schock oder Sepsis, einhergeht, zur Linderung von Schmerzzuständen im allgemeinen oder zu präventiven oder akut therapeutischen Beeinflussung der durch Gefäßerweiterung und erhöhten Blutfluss verursachten Symptomatik von Hitzewallungen menopausaler, östrogendefizienter Frauen sowie hormonbehandelter Prostatakarzinompatienten.

7. Verfahren zur Herstellung eines Arzneimittels gemäß Anspruch 3, **dadurch gekennzeichnet, dass** auf nichtchemischem Weg eine Verbindung nach mindestens einem der Ansprüche 1 bis 2 in einen oder mehrere inerte Trägerstoffe und/oder Verdünnungsmittel eingearbeitet wird.

## Claims

1. Individual CGRP-antagonists of general formula (I) 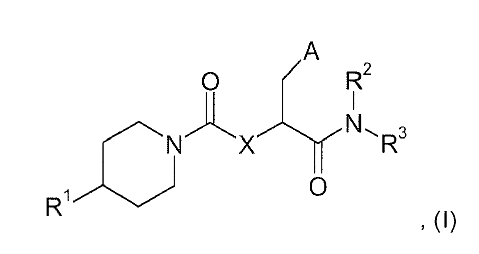 wherein
A denotes a group of formula 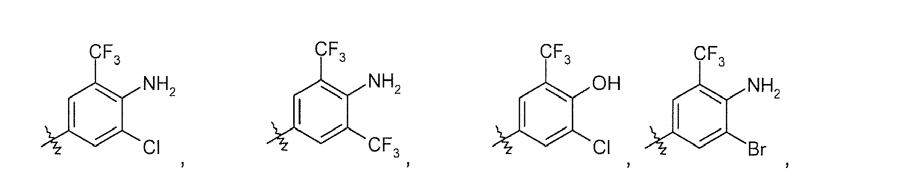 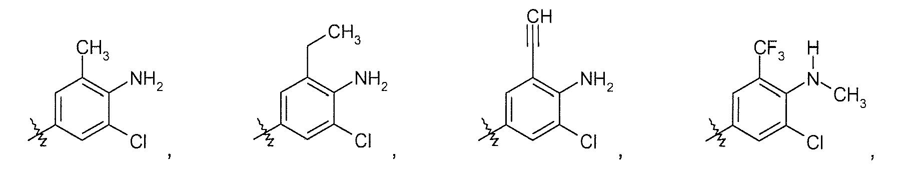 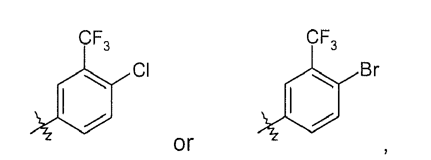 X denotes an oxygen atom, a methylene or NH group,
R¹ denotes a group of formula 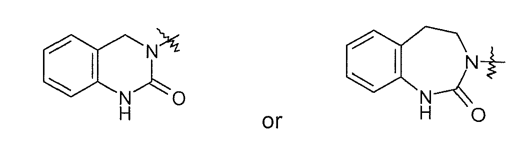 and
-NR²R³ denotes a group of formula 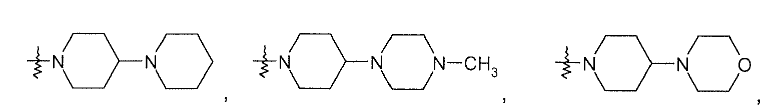 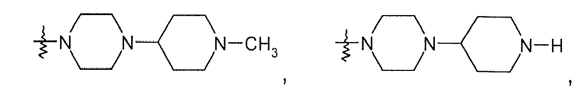 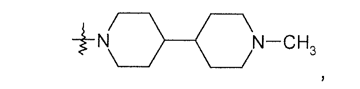 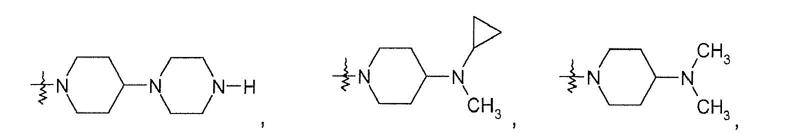 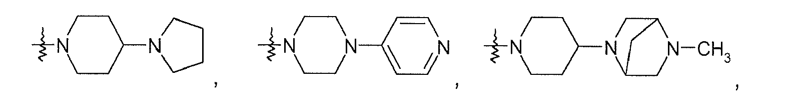 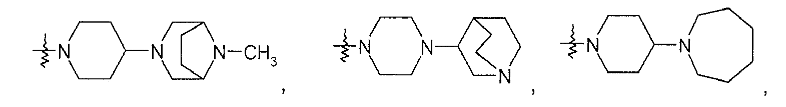 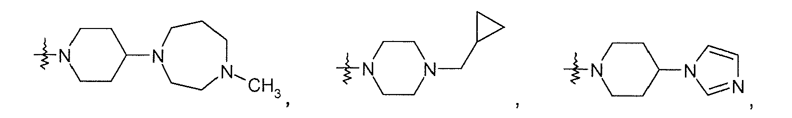 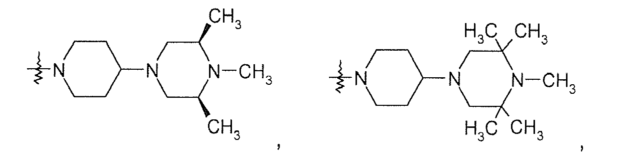 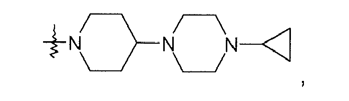 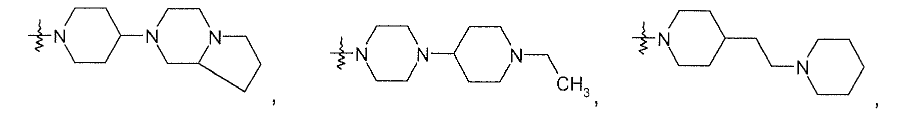 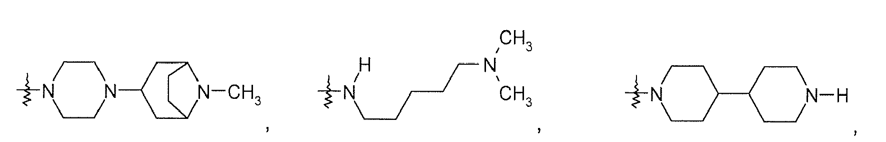 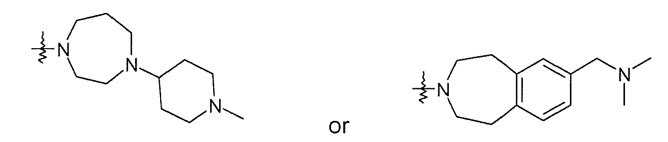 namely:
(1) (*R*)-1-(4-amino-3-chloro-5-trifluoromethyl-benzyl)-2-oxo-2-[4-(2-piperidin-1-yl-ethyl)-piperidin-1-yl]-ethyl 4-(2-oxo-1,2,4,5-tetrahydro-benzo[*d*][1,3]diazepin-3-yl)-piperidine-1-carboxylate,
(2) (*R*)-1-(4-amino-3-chloro-5-trifluoromethyl-benzyl)-2-[4-(1-aza-bicyclo-[2.2.2]oct-3-yl)-piperazin-1-yl]-2-oxo-ethyl 4-(2-oxo-1,2,4,5-tetrahydro-benzo[*d*][1,3]diazepin-3-yl)-piperidine-1-carboxylate,
(3) (*R*)-1-(4-amino-3-chloro-5-trifluoromethyl-benzyl)-2-[4-(5-methyl-2,5-diazabicyclo[2.2.1]hept-2-yl)-piperidin-1-yl]-2-oxo-ethyl 4-(2-oxo-1,2,4,5-tetrahydro-benzo[*d*][1,3]diazepin-3-yl)-piperidine-1-carboxylate,
(4) (*R*)-2-(4-amino-3-chloro-5-trifluoromethyl-phenyl)-1-(5-dimethylaminopentylcarbamoyl)-ethyl 4-(2-oxo-1,2,4,5-tetrahydro-benzo[*d*][1,3]diazepin-3-yl)-piperidine-1-carboxylate,
(5) (*R*)-1-(4-amino-3-chloro-5-trifluoromethyl-benzyl)-2-oxo-2-[4-((3R,5*S*)-3,4,5-trimethyl-piperazin-1-yl)-piperidin-1-yl]-ethyl 4-(2-oxo-1,2,4,5-tetrahydro-benzo[*d*][1,3]diazepin-3-yl)-piperidine-1-carboxylate,
(6) (*R*)-1-(4-amino-3-chloro-5-trifluoromethyl-benzyl)-2-oxo-2-[4-(3,3,4,5,5-pentamethyl-piperazin-1-yl)-piperidin-1-yl]-ethyl 4-(2-oxo-1,2,4,5-tetrahydro-benzo[*d*][1,3]diazepin-3-yl)-piperidine-1-carboxylate,
(7) (*R*)-1-(4-amino-3-chloro-5-trifluoromethyl-benzyl)-2-[4-(4-cyclopropyl-piperazin-1-yl)-piperidin-1-yl]-2-oxo-ethyl 4-(2-oxo-1,2,4,5-tetrahydro-benzo[*d*][1,3]diazepin-3-yl)-piperidine-1-carboxylate,
(8) (*R*)-1-(4-amino-3-chloro-5-trifluoromethyl-benzyl)-2-[4-(1-methyl-piperidin-4-yl)-[1,4]diazepan-1-yl]-2-oxo-ethyl 4-(2-oxo-1,2,4,5-tetrahydro-benzo[*d*][1,3]diazepin-3-yl)-piperidine-1-carboxylate,
(9) (*R*)-1-(4-amino-3-chloro-5-trifluoromethyl-benzyl)-2-(7-dimethylaminomethyl-1,2,4,5-tetrahydro-benzo[*d*]azepin-3-yl)-2-oxo-ethyl 4-(2-oxo-1,2,4,5-tetrahydro-benzo[*d*][1,3]diazepin-3-yl)-piperidine-1-carboxylate,
(10) (*R*)-1-(4-amino-3-chloro-5-trifluoromethyl-benzyl)-2-[4-(cyclopropyl-methyl-amino)-piperidin-1-yl]-2-oxo-ethyl 4-(2-oxo-1,2,4,5-tetrahydro-benzo[*d*][1,3]diazepin-3-yl)-piperidine-1-carboxylate,
(11) (R)-1-(4-amino-3-chloro-5-trifluoromethyl-benzyl)-2-[4-(hexahydro-pyrrolo-[1,2-a]pyrazin-2-yl)-piperidin-1-yl]-2-oxo-ethyl 4-(2-oxo-1,2,4,5-tetrahydro-benzo[*d*][1,3]diazepin-3-yl)-piperidine-1-carboxylate,
(12) (*R*)-1-(4-amino-3-chloro-5-trifluoromethyl-benzyl)-2-[4-(1-ethyl-piperidin-4-yl)-piperazin-1-yl]-2-oxo-ethyl 4-(2-oxo-1,2,4,5-tetrahydro-benzo[*d*][1,3]diazepin-3-yl)-piperidine-1-carboxylate,
(13) (*R*)-1-(4-amino-3-chloro-5-trifluoromethyl-benzyl)-2-[4-(8-methyl-8-azabicyclo[3.2.1]oct-3-yl)-piperazin-1-yl]-2-oxo-ethyl 4-(2-oxo-1,2,4,5-tetrahydro-benzo[*d*][1,3]diazepin-3-yl)-piperidine-1-carboxylate,
(14) (*R*)-1-(4-amino-3-chloro-5-trifluoromethyl-benzyl)-2-[4-(4-methyl-[1,4]diazepan-1-yl)-piperidin-1-yl]-2-oxo-ethyl 4-(2-oxo-1,2,4,5-tetrahydro-benzo[*d*][1,3]diazepin-3-yl)-piperidine-1-carboxylate,
(15) (*R*)-1-(4-amino-3-chloro-5-trifluoromethyl-benzyl)-2-(4-cyclopropylmethyl-piperazin-1-yl)-2-oxo-ethyl 4-(2-oxo-1,2,4,5-tetrahydro-benzo[*d*][1,3]diazepin-3-yl)-piperidine-1-carboxylate,
(16) (*R*)-1-(4-amino-3-chloro-5-trifluoromethyl-benzyl)-2-(4-azepan-1-yl-piperidin-1-yl)-2-oxo-ethyl 4-(2-oxo-1,2,4,5-tetrahydro-benzo[*d*][1,3]diazepin-3-yl)-piperidine-1-carboxylate,
(17) (*R*)-1-(4-amino-3-chloro-5-trifluoromethyl-benzyl)-2-(4-morpholin-4-yl-piperidin-1-yl)-2-oxo-ethyl 4-(2-oxo-1,2,4,5-tetrahydro-benzo[*d*][1,3]diazepin-3-yl)-piperidine-1-carboxylate,
(18) (*R*)-1-(4-amino-3-chloro-5-trifluoromethyl-benzyl)-2-(4-imidazol-1-yl-piperidin-1-yl)-2-oxo-ethyl 4-(2-oxo-1,2,4,5-tetrahydro-benzo[*d*][1,3]diazepin-3-yl)-piperidine-1-carboxylate,
(19) (*R*)-1-(4-chloro-3-trifluoromethyl-benzyl)-2-[4-(4-methyl-piperazin-1-yl)-piperidin-1-yl]-2-oxo-ethyl 4-(2-oxo-1,2,4,5-tetrahydro-benzo[*d*][1,3]diazepin-3-yl)-piperidine-1-carboxylate,
(20) (R)-1-(4-chloro-3-trifluoromethyl-benzyl)-2-[4-(1-methyl-piperidin-4-yl)-piperazin-1-yl]-2-oxo-ethyl 4-(2-oxo-1,2,4,5-tetrahydro-benzo[d][1,3]diazepin-3-yl)-piperidine-1-carboxylate,
the tautomers, the diastereomers, the enantiomers, the hydrates thereof, the mixtures thereof and the salts thereof as well as the hydrates of the salts.

2. Physiologically acceptable salts of the compounds according to claim 1 with inorganic or organic acids.

3. Pharmaceutical compositions containing a compound according to claim 1 or a physiologically acceptable salt according to claim 2, optionally together with one or more inert carriers and/or diluents.

4. Use of a compound according to at least one of claims 1 to 2 for preparing a pharmaceutical composition for the acute and prophylactic treatment of headaches, particularly migraine or cluster headaches.

5. Use of a compound according to at least one of claims 1 to 2 for preparing a pharmaceutical composition for the treatment of non-insulin-dependent diabetes mellitus (NIDDM).

6. Use of a compound according to at least one of claims 1 to 2 for preparing a pharmaceutical composition for the treatment of CRPS1 (complex regional pain syndrome), cardiovascular diseases, morphine tolerance, diarrhoea caused by clostridium toxin, skin diseases, particularly thermal and radiation-induced skin damage including sunburn, inflammatory diseases, such as, in particular, inflammatory diseases of the joints such as arthritis, neurogenic inflammation of the oral mucosa, inflammatory lung diseases, allergic rhinitis, asthma, diseases accompanied by excessive vasodilatation and resultant reduced circulation of the blood, e.g. shock and sepsis, for alleviating pain in general or for preventive or acute therapeutic treatment of the symptoms of hot flushes caused by vasodilatation and increased blood flow in menopausal, oestrogen-deficient women and hormone-treated patients with prostate carcinoma.

7. Process for preparing a pharmaceutical composition according to claim 3, **characterised in that** a compound according to at least one of claims 1 to 2 is incorporated in one or more inert carriers and/or diluents by a non-chemical method.

## Revendications

1. Antagonistes de CGRP individuels de formule générale (I) 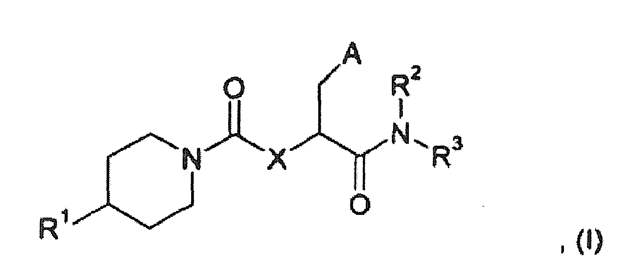 dans laquelle
A est un radical de formule 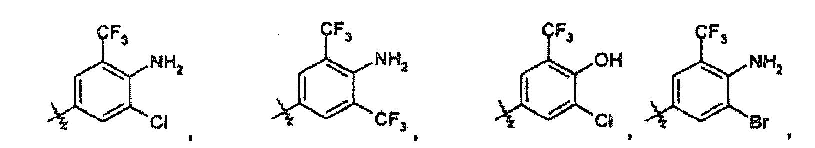 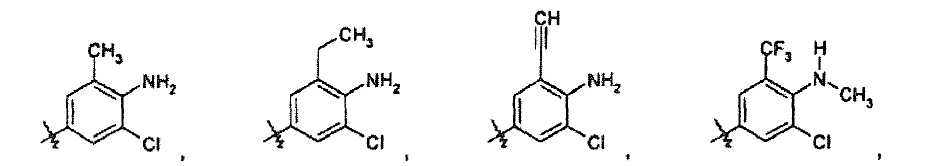 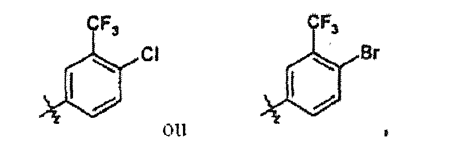 X est un atome d'oxygène, un groupe méthylène ou un groupe NH,
R¹ est un radical de formule 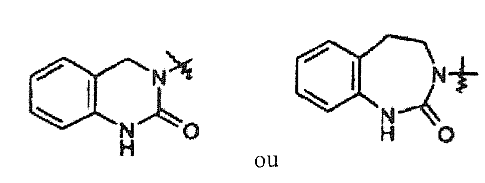 et
-NR²R³ est un radical de formule 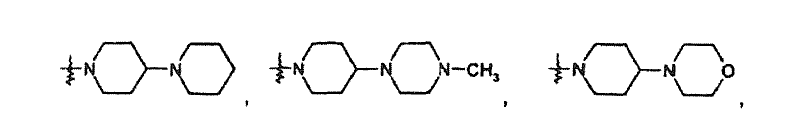 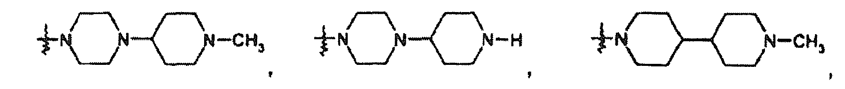 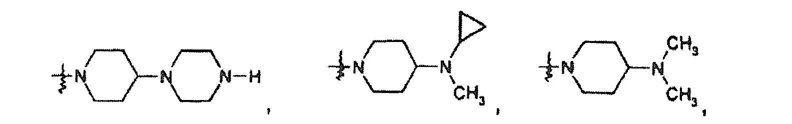 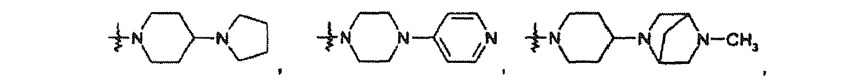 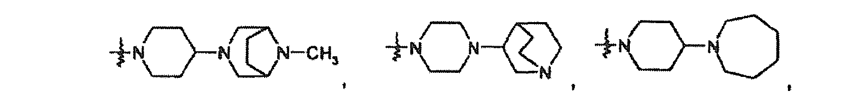 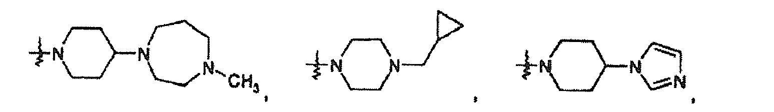 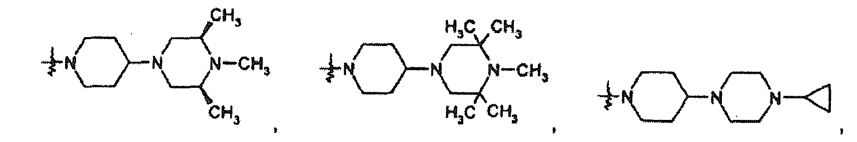 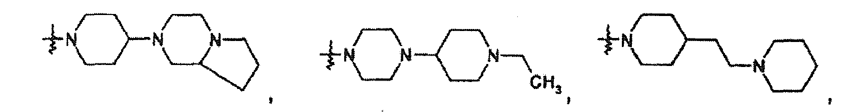 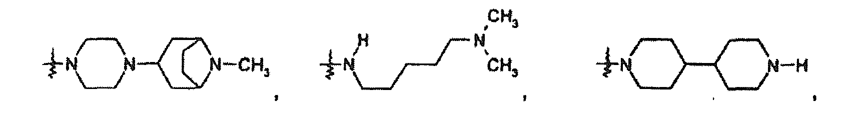 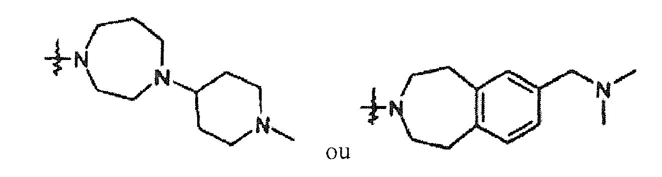 à savoir, les :
(1) acide 4-(2-oxo-1,2,4,5-tétrahydro-benzo[*d*][1,3]diazépin-3-yl)-pipéridin-1-carboxylique-(*R*)-1-(4-amino-3-chloro-5-trifluorométhyl-benzyl)-2-oxo-2-[4-(2-pipéridin-1-yl-éthyl)-pipéridin-1-yl]-éthylester,
(2) acide 4-(2-oxo-1,2,4,5-tétrahydro-benzo[*d*][1,3]diazépin-3-yl)-pipéridin-1-carboxylique-(*R*)-1-(4-amino-3-chloro-5-trifluorométhyl-benzyl)-2-[4-(1-azabicyclo-[2.2.2]oct-3-yl)-pipérazin-1-yl]-2-oxo-éthylester,
(3) acide 4-(2-oxo-1,2,4,5-tétrahydro-benzo[*d*][1,3]diazépin-3-yl)-pipéridin-1-carboxylique-(*R*)-1-(4-amino-3-chloro-5-trifluorométhyl-benzyl)-2-[4-(5-méthyl-2,5-diaza-bieyclo[2.2.1]hept-2-yl)-pipéridin-1-yl]-2-oxo-éthylester,
(4) acide 4-(2-oxo-1,2,4,5-tétrahydro-benzo[*d*][1,3]diazépin-3-yl)-pipéridin-1-earboxylique-(*R*)-2-(4-amino-3-chloro-5-trifluorométhyl-phényl)-1-(5-diméthylamino-pentylcarbamoyl)-éthylester,
(5) acide 4-(2-oxo-1,2,4,5-tétrahydro-benzo[*d*][1,3]diazépin-3-yl)-pipéridin-1-carboxylique-(*R*)-1-(4-amino-3-chloro-5-trifluorométhyl-benzyl)-2-oxo-2-[4-((3*R-*5*S*)-3,4,5-triméthyl-pipérazin-1-yl)-pipéridin-1-yl]-éthylester,
(6) acide 4-(2-oxo-1,2,4,5-tétrahydro-benzo[*d*][1,3]diazépin-3-yl)-pipéridin-l-carboxylique-(*R*)-1-(4-amino-3-chloro-5-trifluorométhyl-benzyl)-2-oxo-2-[4-(3,3,4,5,5-pentaméthyl-pipérazin-1-yl)-pipéridin-1-yl]-éthylester,
(7) acide 4-(2-oxo-1,2,4,5-tétrahydro-benzo[*d*][1,3]diazépin-3-yl)-pipéridin-1-carboxylique-(*R*)-1-(4-amino-3-chloro-5-trifluorométhyl-benzyl)-2-[4-(4-cyclopropyl-pipérazin-1-yl)-pipéridin-1-yl]-2-oxo-éthylester,
(8) acide 4-(2-oxo-1,2,4,5-tétrahydro-benzo[*d*][1,3]diazépin-3-yl)-pipéridin-1-carboxylique-(*R*)-1-(4-amino-3-chloro-5-trifluorométhyl-bcnzyl)-2-[4-(1-méthyl-pipéridin-4-yl)-[1,4]diazépan-1-yl]-2-oxo-éthylester,
(9) acide 4-(2-oxo-1,2,4,5-tétrahydro-benzo[*d*][1,3]diazépin-3-yl)-pipéridin-1-carboxylique-(*R*)-1-(4-amino-3-chloro-5-trifluorométhyl-benzyl)-2-(7-diméthylamino-méthyl-1,2,4,5-tétrahydro-benzo[d]azépin-3-yl)-2-oxo-éthylester,
(10) acide 4-(2-oxo-1,2,4,5-tétrahydro-benzo[*d*][1,3]diazépin-3-yl)-pipéridin-1-carboxylique-(*R*)-1-(4-amino-3-chloro-5-trifluorométhyl-benzyl)-2-[4-(cyclopropyl-méthyl-amino)-pipéridin-1-yl]-2-oxo-éthylester,
(11) acide 4-(2-oxo-1,2,4,5-tétrahydro-benzo[*d*][1,3]diazépin-3-yl)-pipéridin-1-carboxylique-(*R*)-1-(4-amino-3-chloro-5-trifluorométhyl-benzyl)-2-[4-(hexahydro-pyrrolo-[1,2-a]pyrazin-2-yl)-pipéridin-1-yl]-2-oxo-éthylester,
(12) acide 4-(2-oxo-1,2,4,5-tétrahydro-benzo[*d*][1,3]diazépin-3-yl)-pipéridin-1-carboxylique-(*R*)-1-(4-amino-3-chloro-5-trifluorométhyl-benzyl)-2-[4-(1-éthyl-pipéridin-4-yl)-pipérazin-1-yl]-2-oxo-éthylester,
(13) acide 4-(2-oxo-1,2,4,5-tétrahydro-benzo[*d*][1,3]diazépin-3-yl)-pipéridin-1-carboxylique-(*R*)-1-(4-amino-3-chloro-5-trifluorométhyl-benzyl)-2-[4-(8-méthyl-8-aza-bicyclo[3.2.1]oct-3-yl)-pipérazin-1-yl]-2-oxo-éthylester,
(14) acide 4-(2-oxo-1,2,4,5-tétrahydro-benzo[*d*][1,3]diazépin-3-yl)-pipéridin-1-carboxylique-(*R*)-1-(4-amino-3-chloro-5-trifluorométhyl-benzyl)-2-[4-(4-méthyl-[1,4]diazépan-1-yl)-pipéridin-1-yl]-2-oxo-éthylester,
(15) acide 4-(2-oxo-1,2,4,5-tétrahydro-benzo[*d*][1,3]diazépin-3-yl)-pipéridin-1-carboxylique-(*R*)-1-(4-amino-3-chloro-5-trifluorométhyl-benzyl)-2-(4-cyclopropylméthyl-pipérazin-1-yl)-2-oxo-éthylester,
(16) acide 4-(2-oxo-1,2,4,5-tétrahydro-benzo[*d*][1,3]diazépin-3-yl)-pipéridin-1-carboxylique-(*R*)-1-(4-amino-3-chloro-5-trifluorométhyl-bcnzyl)-2-(4-azépan-1-yl-pipéridin-1-yl)-2-oxo-éthylester,
(17) acide 4-(2-oxo-1,2,4,5-tétrahydro-benzo[*d*][1,3]diazépin-3-yl)-pipéridin-1-carboxylique-(*R*)-1-(4-amino-3-chloro-5-trifluorométhyl-benzyl)-2-(4-morpholin-4-yl-pipéridin-1-yl)-2-oxo-éthylester,
(18) acide 4-(2-oxo-1,2,4,5-tétrahydro-benzo[*d*][1,3]diazépin-3-yl)-pipéridin-1-carboxylique-(*R*)-1-(4-amino-3-chloro-5-trifluorométhyl-bcnzyl)-2-(4-imidazol-1-yl-pipéridin-1-yl)-2-oxo-éthylester,
(19) acide 4-(2-oxo-1,2,4,5-tétrahydro-benzo[*d*][1,3]diazépin-3-yl)-pipéridin-1-carboxylique-(*R*)-1-(4-chloro-3-trifluorométhyl-benzyl)-2-[4-(4-méthyl-pipérazin-1-yl)-pipéridin-1-yl]-2-oxo-éthylester,
(20) acide 4-(2-oxo-1,2,4,5-tétrahydro-benzo[*d*][1,3]diazépin-3-yl)-pipéridin-1-carboxylique-(*R*)-1-(4-chloro-3-trifluorométhyl-benzyl)-2-[4-(1-méthyl-pipéridin-4-yl)-pipérazin-1-yl]-2-oxo-éthylester,
leurs tautomères, leurs diastéréomères, leurs énantiomères, leurs hydrates, leurs mélanges et leurs sels et les hydrates des sels.

2. Sels physiologiquement acceptables des composés selon la revendication 1 avec des acides inorganiques ou organiques.

3. Médicament contenant un composé selon la revendication 1 ou un sel physiologiquement acceptable selon la revendication 2, et éventuellement un ou plusieurs véhicules et/ou diluants inertes.

4. Utilisation d'un composé selon au moins l'une des revendications 1 à 2, pour la préparation d'un médicament pour le traitement aigu ou prophylactique des céphalées, en particulier, des migraines ou des céphalées de type algies vasculaires de la face.

5. Utilisation d'un composé selon au moins l'une des revendications 1 à 2, pour la préparation d'un médicament pour lutter contre le diabète sucré non insulino-dépendant (NIDDM).

6. Utilisation d'un composé selon au moins l'une des revendications 1 à 2, pour la préparation d'un médicament pour le traitement du CRPS1 (syndrome de douleur régionale complexe), des maladies cardiovasculaires, de la tolérance à la morphine, des diarrhées dues à la toxine *Chlostridium,* de maladies de la peau, en particulier, des lésions thermiques et dues au rayonnement, y compris les coups de soleil, des maladies inflammatoires telles que, en particulier, des maladies articulaires inflammatoires telles que l'arthrite, des inflammations neurogènes de la muqueuse buccale, des maladies pulmonaires inflammatoires, de la rhinite allergique, de l'asthme, de maladies qui s'accompagnent d'un élargissement vasculaire excessif et ainsi, d'une réduction de l'irrigation vasculaire, telles qu'en particulier, un état de choc ou la septicémie, pour calmer les douleurs en général ou pour un effet préventif ou thérapeutique en phase aiguë des symptômes de bouffées de chaleur provoqués par la dilatation vasculaire et l'augmentation du débit sanguin chez les femmes ménopausées présentant une déficience en oestrogène et les patients souffrant de carcinome de la prostate traités par des hormones.

7. Procédé de préparation d'un médicament selon la revendication 3, **caractérisé en ce qu'**un composé est intégré de façon non chimique selon au moins l'une des revendications 1 à 2, dans un ou plusieurs véhicule(s) et/ou diluant(s) inerte(s).
